# EUROPEAN PATENT SPECIFICATION

(11) **EP 2 623 017 B1**
(45) Date of publication and mention of the grant of the patent: **08.11.2017**
(21) Application number: 12767417.4
(22) Date of filing: 02.04.2012
(51) Int. Cl.: A61B 1/00, A61B 1/04, A61B 5/00

(54) **RECEIVING DEVICE AND CAPSULE ENDOSCOPE SYSTEM**
EMPFANGSVORRICHTUNG UND KAPSELENDOSKOPSYSTEM
DISPOSITIF DE RÉCEPTION ET SYSTÈME D'ENDOSCOPE À CAPSULE

(30) Priority: 01.04.2011 JP 2011082297
(43) Date of publication of application: 07.08.2013
(73) Proprietor: Olympus Corporation, Tokyo 192-8507 (JP)
(72) Inventor: TANIGUCHI, Katsuyoshi, Hachioji-shi, Tokyo 192-8507 (JP)
(74) Representative: Gunzelmann, Rainer
(86) International application number: PCT/JP2012/058832
(87) International publication number: WO 2012/137705

(56) References cited:
- EP-A1- 2 140 799
- WO-A1-2006/028047
- WO-A1-2008/105369
- JP-A- 2004 362 370
- JP-A- 2005 342 905
- JP-A- 2006 509 574
- JP-A- 2009 081 752
- US-A1- 2009 076 326
- US-A1- 2009 203 964

## Description

### Field

The present invention relates to a receiving apparatus that receives image information and the like, which are transmitted wirelessly from a capsule endoscope inserted into a subject, and a capsule endoscope system. Background

In an examination using a capsule endoscope inserted into a subject and capturing an in-vivo image of the subject, the image data acquired by the capsule endoscope and transmitted wirelessly are received by a receiving apparatus attached to the outside of the body of the subject. The image data received by the receiving apparatus are accumulated in memory built into the receiving apparatus during the examination, transferred (downloaded) to an image observation apparatus such as a workstation via a cradle after the end of the examination, and used for a diagnosis by a doctor.

The receiving apparatus is handled by both of a health care professional such as a nurse, and a patient (subject) in a series of flows of the capsule endoscopy. For example, at an examination preparation stage, the health care professional performs operations such as the initialization of the built-in memory of the receiving apparatus, registration of patient information, and a check on the reception of a wireless signal from the capsule endoscope. When the examination subsequently starts, the patient is given time to act substantially freely in a state of carrying the receiving apparatus. Furthermore, when reaching at the final stage of the examination, the health care professional performs operations such as the initialization of the built-in memory of the receiving apparatus, registration of patient information, and a check on the reception of a wireless signal from the capsule endoscope. When the examination subsequently starts, the patient is given time to act substantially freely in a state of carrying the receiving apparatus. Furthermore, when reaching at the final stage of the examination, the health care professional performs operations, such as confirmation of the end of the examination, on the receiving apparatus. The receiving apparatus is subsequently removed from the patient, and the health care professional downloads the image data (for example, refer to patent literature 1).

### Citation List

### Patent Literature

### Patent Literature 1: Japanese Laid-open Patent Publication No. 2007-175446

US 2009/203964 discloses a capsule endoscope system including a capsule endoscope and a receiver for wirelessly receiving images from the capsule endoscope, according to the preamble of claim 1. US 2009/076326 discloses a capsule endoscope having a plurality of operation modes correlated to a location in the subject through which the capsule endoscope passes. A storage location of images is different for each operation mode and an imaging interval changes based on the location of the capsule in the body. Additional imaging parameters may be associated with an operating mode, such as a baseline luminance, a gain control, etc.

EP 2 140 799 discloses a capsule medical device in which information such as patient identification is transferred to a server, which further receives a diagnosis result obtained from an examination using the capsule medical device. The capsule system is prevented from transferring images to a server which does not comprise the patient information.

A receiving apparatus and a capsule endoscope system according to the appended independent claims are provided.

### Summary

### Technical Problem

As described above, because one receiving apparatus of a capsule endoscope system is usable by two different types of users who are in different positions, i.e., a health care professional and a patient, there is the possibility that a user may execute a wrong function for other user instead of a function for him/her. If, especially, a patient operates it carelessly during his/her free action time, the examination may fail due to an operating error.

The present invention has been made to solve the above problem and it is an object of the present invention to provide, regarding a receiving apparatus that is commonly used by a health care professional and a patient, a receiving apparatus and a capsule endoscope system capable of preventing occurrence of an operating error. Solution to Problem To solve the above problem and achieve the above object, a receiving apparatus according to the present invention receives information transmitted wirelessly from a capsule endoscope for inserted into a subject and capturing an in-vivo image of the subject. The receiving apparatus includes an operation input unit that accepts input of information in the receiving apparatus; a display unit that displays information related to an examination using the capsule endoscope; and a control unit that controls operations of the receiving apparatus. The control unit includes an examination flow management unit that controls a flow of a series of processes in the examination, and an operation mode setting unit that switches an operation mode of the receiving apparatus between a first operation mode and a second operation mode being different from the first operation mode in accordance with progress of the examination. The control unit controls the operations of the receiving apparatus in accordance with progress of the examination for each operation mode set by the operation mode setting unit.

In the above receiving apparatus, the examination flow management unit controls the flow of the series of processes based on examination flow information in which each of the processes is associated with either the first operation mode or the second operation mode.

In the above receiving apparatus, the first operation mode is an operation mode for a health care professional, and the control unit displays on the display unit information related to a process to be executed or confirmed by the health care professional when the receiving apparatus is set in the first operation mode.

In the above receiving apparatus, when the operation input unit performs operation input in accordance with progress of the examination when the receiving apparatus is set in the first operation mode, the examination flow management unit permits a shift to a process corresponding to the operation input.

In the above receiving apparatus, the first operation mode is an operation mode for a health care professional, and the operation mode setting unit switches the operation mode of the receiving apparatus to the second operation mode in accordance with progress of the examination when the receiving apparatus is set in the first operation mode.

In the above receiving apparatus, the second operation mode is an operation mode for a patient, and when the operation input unit performs operation input other than previously permitted operation input when the receiving apparatus is set in the second operation mode, the control unit disables the operation input.

In the above receiving apparatus, the second operation mode is an operation mode for a patient, and the control unit displays instruction information to the patient on the display unit in accordance with progress of the examination when the receiving apparatus is set in the second operation mode.

In the above receiving apparatus, the second operation mode is an operation mode for a patient, and the operation mode setting unit switches the operation mode of the receiving apparatus to the first operation mode when the operation input unit performs predetermined operation input when the receiving apparatus is set in the second operation mode.

A capsule endoscope system according to the present invention includes a capsule endoscope; the above receiving apparatus; and a control apparatus for transmitting and receiving information in wired or wireless communication to and from the receiving apparatus.

Moreover, a capsule endoscope system according to the present invention includes a capsule endoscope for being inserted into a subject and capturing an in-vivo image of the subject; a receiving apparatus for receiving information transmitted wirelessly from the capsule endoscope; and a control apparatus for transmitting and receiving information in wired or wireless communication to and from the receiving apparatus. The receiving apparatus includes an operation input unit that accepts input of information in the receiving apparatus, a display unit that displays information related to an examination using the capsule endoscope, and a first control unit that controls operations of the receiving apparatus. The control unit includes an examination flow management unit that controls a flow of a series of processes in the examination based on progress of the examination, and a second control unit generates control information in accordance with progress of the examination and transmits the control to the receiving apparatus. The first control unit includes an operation mode setting unit that switches the operation mode of the receiving apparatus between a first operation mode and a second operation mode being different from the first operation mode in accordance with the control information, and controls an operation of the receiving apparatus in accordance with the control information for each operation mode set by the operation mode setting unit.

In the above capsule endoscope system, the first control unit transmits information about progress of the examination in the receiving apparatus from the receiving apparatus to the control apparatus.

In the above capsule endoscope system, the examination flow management unit controls the flow of the series of processes based on examination flow information in which each of the processes is associated with either the first operation mode or the second operation mode.

In the above capsule endoscope system, the first operation mode is an operation mode for a health care professional, and the first control unit displays on the display unit information related to a process to be executed or confirmed by the health care professional when the receiving apparatus is set in the first operation mode.

In the above capsule endoscope system, when the operation input unit performs operation input in accordance with progress of the examination when the receiving apparatus is set in the first operation mode, the first control unit permits a shift to a process corresponding to the operation input.

In the above capsule endoscope system, the first operation mode is an operation mode for a health care professional, and the operation mode setting unit switches to the second operation mode in accordance with progress of the examination when the receiving apparatus is set in the first operation mode.

In the above capsule endoscope system, the second operation mode is an operation mode for a patient, and when the operation input unit performs operation input other than previously permitted operation input when the receiving apparatus is set in the second operation mode, the first control unit disables the operation input.

In the above capsule endoscope system, the second operation mode is an operation mode for a patient, and the first control unit displays instruction information to the patient on the display unit in accordance with progress of the examination when the receiving apparatus is set in the second operation mode.

In the above capsule endoscope system, the second operation mode is an operation mode for a patient, and the operation mode setting unit switches the operation mode of the receiving apparatus to the first operation mode when the operation input unit performs predetermined operation input or when the receiving apparatus receives predetermined control information when the receiving apparatus is set in the second operation mode.

### Advantageous Effects of Invention

According to the present invention, the operation mode is switched between a first operation mode and a second operation mode being different from the first operation mode in accordance with progress of the examination; therefore, it is possible to prevent receiving any operation that is out of a permitted range of each process of a flow of an examination.

### Brief Description of Drawings

FIG. 1 is a schematic diagram illustrating the schematic configuration of a capsule endoscope system according to a first embodiment of the present invention.
FIG. 2 is a front view illustrating the appearance of a receiving apparatus illustrated in FIG. 1.
FIG. 3 is a block diagram illustrating the configuration of the receiving apparatus illustrated in FIG. 1.
FIG. 4 is a table illustrating examination flow information.
FIG. 5 is a flowchart illustrating the operations of the capsule endoscope system illustrated in FIG. 1.
FIG. 6 is a flowchart illustrating the operations of the receiving apparatus and a control apparatus at the examination preparation stage illustrated in FIG. 5.
FIG. 7A is a display example of a screen of the receiving apparatus at the examination preparation stage.
FIG. 7B is a display example of a screen for patient information registration of when patient information is registered on the receiving apparatus side.
FIG. 7C is a display example of screens of the receiving apparatus at the examination preparation stage.
FIG. 7D is a display example of screens of the receiving apparatus at the examination preparation stage.
FIG. 7E is a display example of screens of the receiving apparatus at the examination preparation stage.
FIG. 7F is a display example of screens of the receiving apparatus at the examination preparation stage.
FIG. 7G is a display example of screens of the receiving apparatus at the examination preparation stage.
FIG. 7H is a display example of screens of the receiving apparatus at the examination preparation stage.
FIG. 7I is a display example of screens of the receiving apparatus at the examination preparation stage.
FIG. 7J is a display example of screens of the receiving apparatus at the examination preparation stage.
FIG. 7K is a display example of screens of the receiving apparatus at the examination preparation stage.
FIG. 7L is a display example of screens of the receiving apparatus at the examination preparation stage.
FIG. 7M is a display example of screens of the receiving apparatus at the examination preparation stage.]
FIG. 7N is a display example of screens of the receiving apparatus at the examination preparation stage.
FIG. 8 is a flowchart illustrating the operations of the receiving apparatus at the examination stage illustrated in FIG. 5.
FIG. 9A is a display example of screens of the receiving apparatus at the examination stage.
FIG. 9B is a display example of screens of the receiving apparatus at the examination stage.
FIG. 9C is a display example of screens of the receiving apparatus at the examination stage.
FIG. 9D is a display example of screens of the receiving apparatus at the examination stage.
FIG. 9E is a display example of a revisit screen.
FIG. 10 is a flowchart illustrating the operations of the receiving apparatus at the post-examination processing stage illustrated in FIG. 5.
FIG. 11A is a view illustrating a password input screen.
FIG. 11B is a display example of screens of the receiving apparatus at the post-examination processing stage.
FIG. 11C is a display example of screens of the receiving apparatus at the post-examination processing stage.
FIG. 11D is a display example of screens of the receiving apparatus at the post-examination processing stage.
FIG. 11E is a display example of screens of the receiving apparatus at the post-examination processing stage.
FIG. 11F is a display example of screens of the receiving apparatus at the post-examination processing stage.
FIG. 11G is a display example of screens of the receiving apparatus at the post-examination processing stage.
FIG. 12A is a view for explaining a method for displaying the password input screen.
FIG. 12B is a view illustrating a modification of a method for inputting a password.
FIG. 12C is a view illustrating another modification of the method for inputting the password.
FIG. 13 is a schematic diagram illustrating a configuration example of the system in Modification 1-3.
FIG. 14A is a display example of screens of the receiving apparatus at the examination stage.
FIG. 14B is a display example of screens of the receiving apparatus at the examination stage.
FIG. 15 is a schematic diagram illustrating a configuration example of a download device according to Modification 1-5.
FIG. 16 is a schematic diagram illustrating a configuration example of a download system.
FIG. 17 is a schematic diagram illustrating another configuration example of the download system.
FIG. 18 is a display example of a screen of the receiving apparatus in Modification 1-6.
FIG. 19A is a display example of an examination report.
FIG. 19B is a table illustrating statistic information of examination implementation information.
FIG. 20 is a block diagram illustrating the configuration of a receiving apparatus according to a second embodiment of the present invention.
FIG. 21 is a flowchart illustrating operations at the examination preparation stage of the receiving apparatus illustrated in FIG. 20.
FIG. 22 is a display example of a screen to notify the passage of a capsule endoscope through the stomach.
FIG. 23 is a display example of a screen to notify the arrival of the capsule endoscope at the large intestine in Modification 2-1.
FIG. 24 is a block diagram illustrating the configuration of a receiving apparatus according to a third embodiment of the present invention.
FIG. 25 is a table illustrating abnormality detection information.
FIG. 26 is a display example of an action instruction screen for a patient.
FIG. 27 is a schematic diagram illustrating the schematic configuration of a capsule endoscope system according to a fourth embodiment of the present invention.
FIG. 28 is a block diagram illustrating the configuration of the capsule endoscope system illustrated in FIG. 27.
FIG. 29 is a flowchart illustrating the operations of the receiving apparatus and the control apparatus, which are illustrated in FIG. 28.
FIG. 30 is a table illustrating examination management information.
FIG. 31 is a block diagram illustrating the configuration of a capsule endoscope system according to a fifth embodiment of the present invention.
FIG. 32A is a display example of a screen to notify the occurrence of an abnormality.
FIG. 32B is a display example of a screen of a message to the patient.
FIG. 33A is a display example of a screen to instruct the patient on action.
FIG. 33B is a display example of a reason input screen.
FIG. 34A is a display example of a screen during the examination.
FIG. 34B is a display example of a question input screen.
FIG. 34C is a display example of a question display screen.
FIG. 34D is a display example of an answer display screen.
FIG. 35 is a block diagram illustrating the configuration of a capsule endoscope system according to a sixth embodiment of the present invention.
FIG. 36 is a flowchart illustrating processes of confirming the identity of the patient.
FIG. 37A is a display example of a screen to give an instruction to capture an image of the patient's face.
FIG. 37B is a display example of a screen to notify the completion of the confirmation of the identity.
FIG. 37C is a display example of a screen to notify the incompletion of the confirmation of the identity.
FIG. 38 is a block diagram illustrating the configuration of a capsule endoscope system according to a seventh embodiment.
FIG. 39 is a table illustrating capsule inventory management information.
FIG. 40A is a display example of a screen to notify the quantity of the inventory of the capsule endoscope.
FIG. 40B is a display example of a screen to warn of a reduction in the quantity of the inventory of the capsule endoscope.
FIG. 40C is a display example of a screen to notify the expiration date of the capsule endoscope.
FIG. 41 is a table illustrating receiving apparatus management information.
FIG. 42A is a display example of a screen to notify the availability of the receiving apparatuses.
FIG. 42B is a display example of a screen to instruct the replacement of a battery of the receiving apparatus and.
FIG. 42C is a display example of a screen to instruct the charge of the battery of the receiving apparatus.

### Description of Embodiments

A description will hereinafter be given of a receiving apparatus and a capsule endoscope system according to embodiments of the present invention with reference to the drawings. In the following description, a system including a capsule endoscope that is inserted into the body of a subject and captures an in-vivo image is given as an example. However, the present invention is not limited to the embodiments.

### (First Embodiment)

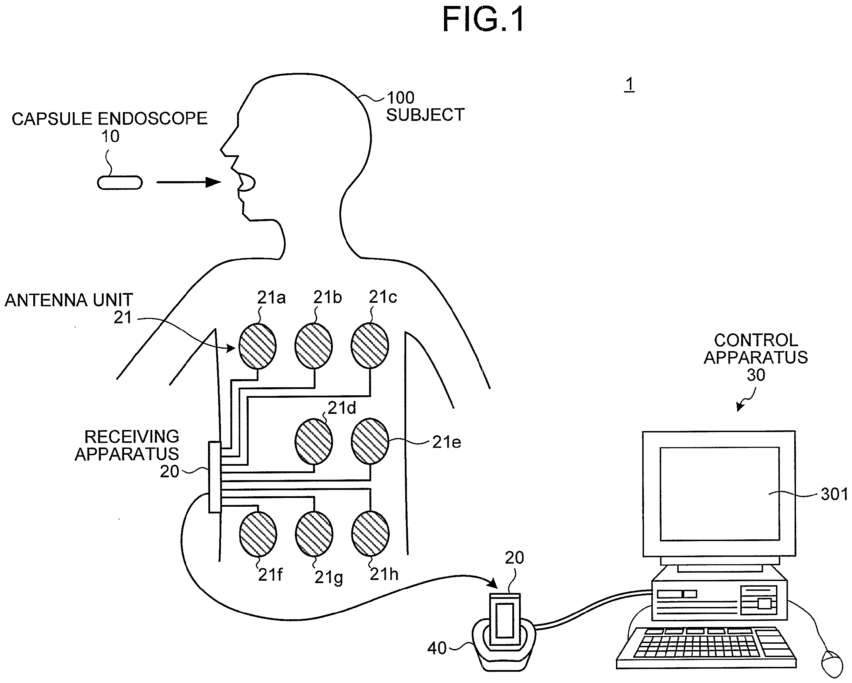

FIG. 1 is a schematic diagram illustrating the schematic configuration of a capsule endoscope system according to a first embodiment of the present invention. This capsule endoscope system 1 includes a capsule endoscope 10 that is inserted into the body of a subject (patient) 100, and wirelessly transmits image data of in-vivo images acquired by capturing the images, a receiving apparatus 20 that receives the image data transmitted wirelessly from the capsule endoscope 10, and a control apparatus 30 that displays the in-vivo images based on the image data transferred from the receiving apparatus 20 via a cradle 40.

The capsule endoscope 10 includes therein various parts such as a lighting device that illuminates inside the subject 100, a condenser lens that condenses light reflected from inside the subject 100, an imaging device, such as a CCD, that converts the received light into an electrical signal (imaging signal), an IC configuring a signal processing unit that processes the imaging signal acquired by the imaging device, and a wireless transmitting antenna. After being swallowed from the mouth of the subject 100, the capsule endoscope 10 successively capture images of body regions (esophagus, stomach, small intestine, large intestine, and the like) at predetermined time intervals (e.g., 0.5-second intervals) while moving in the digestive tract of the subject 100 by the peristaltic movement of the organs. A/D conversion and predetermined signal processing are performed on the acquired imaging signals to generate image data, and the image data, together with related information, are successively transmitted wirelessly to the receiving apparatus 20. The related information contains identification information (e.g., a serial number) assigned to identify an individual of the capsule endoscope 10, and the like.

The receiving apparatus 20 is attached in the vicinity of the body surface of the subject 100, and receives the image data and related information, which have been transmitted wirelessly from the capsule endoscope 10 via an antenna unit 21 including a plurality of (eight in FIG. 1) receiving antennas 21a to 21h. The receiving antennas 21a to 21h are realized by using loop antennas, for example, and arranged at predetermined positions (e.g., a position corresponding to each organ in the subject 100, which is a passage route of the capsule endoscope 10) on the body surface of the subject 100.

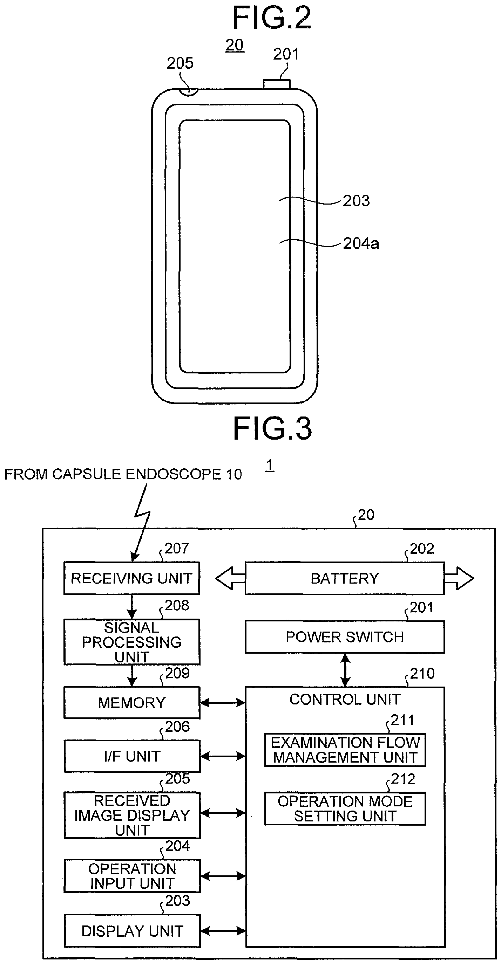

FIG. 2 is a schematic diagram illustrating the appearance of the receiving apparatus 20. Moreover, FIG. 3 is a block diagram illustrating the configuration of the receiving apparatus 20. As illustrated in FIG. 2 and FIG. 3, the receiving apparatus 20 includes a power switch 201 that switches the power states (ON/OFF) of the receiving apparatus 20 by a predetermined operation (e.g., a long press for a predetermined time or more), a battery 202 that supplies power to the units of the receiving apparatus 20, a display unit 203 that displays various information related to an examination, a touch panel 204a as an operation input unit 204 provided by being superimposed on the display unit 203, a received image display unit 205, an interface (I/F) unit 206 that mediates communication with an external device to be connected to the receiving apparatus 20, a receiving unit 207 that receives image data transmitted wirelessly from the capsule endoscope 10 via the antenna unit 21, a signal processing unit 208 that performs predetermined signal processing on the received image data, a memory 209, and a control unit 210. Moreover, the receiving apparatus 20 may include a plurality of hardware operation members such as a push-button as the operation input unit 204, apart from the touch panel 204a.

The display unit 203 is realized by a display panel such as a liquid crystal or organic EL (Electro Luminescence).

The touch panel 204a is an operation input device that detects a position and path of contact with information and images (an icon and the like), which are displayed on the display panel of the display unit 203, and accepts the input of an operation in accordance with the contact position and path. There are systems such as resistive, capacitive, and optical systems for the touch panel. However, any system can be adopted in the first embodiment. The received image display unit 205 includes a lighting device such as an LED, blinks while the receiving apparatus 20 is receiving image data from the capsule endoscope 10, and displays to be in a state where image data is being received.

The memory 209 stores image data of in-vivo images on which signal processing is performed by the signal processing unit 208, information related to a relevant examination (patient information, ID information of the receiving apparatus 20 itself, and the like), programs for controlling the operations of the receiving apparatus 20, and the like. In the first embodiment, a built-in memory is used as the memory 209. However, instead thereof, a memory removable from the receiving apparatus 20, such as USB memory and CompactFlash (registered trademark) may be used.

The control unit 210 is realized by hardware such as a CPU, and reads various programs stored in the memory 209 to integrally control the operations of the entire receiving apparatus 20 in accordance with various operation signals and the like, which are inputted via the interface unit 206. Specifically, the control unit 210 includes an examination flow management unit 211 that controls a flow of a series of processes (examination flow) in an examination using the capsule endoscope 10 (hereinafter may be simply referred to as the examination), and an operation mode setting unit 212 that switches the operation mode of the receiving apparatus 20 between a health care professional mode and a patient mode in accordance with the progress of the examination.

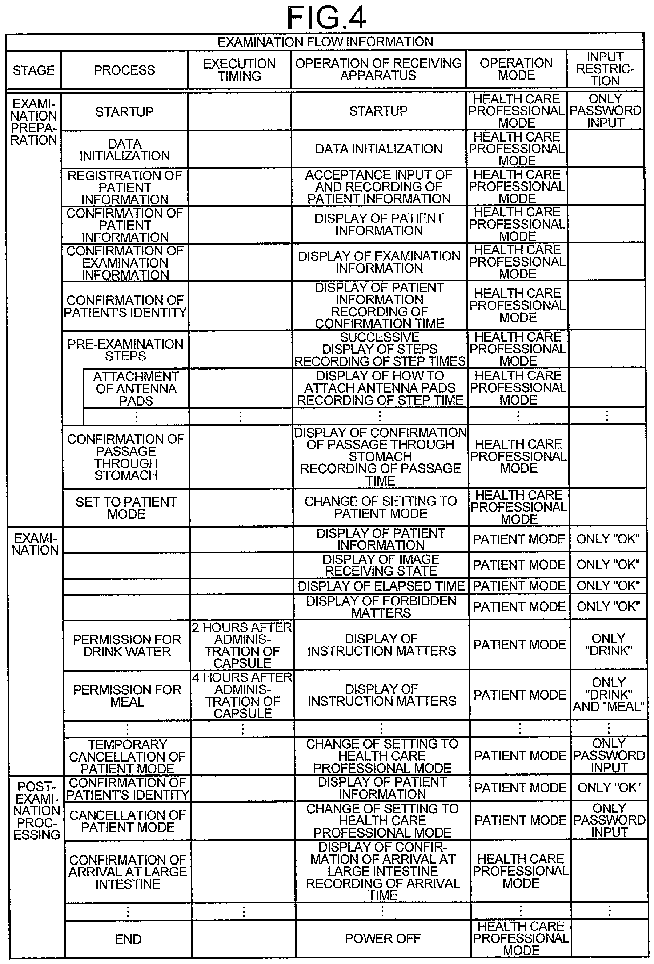

The examination flow management unit 211 controls the flow of processes in the examination in accordance with examination flow information stored in the memory 209. The examination flow information is information where processes at stages of the examination are described. As illustrated in FIG. 4, the examination flow information contains information related to the contents of a series of processes (process contents) that are executed or confirmed by a health care professional or patient at each stage and their execution timings, the contents of an operation (operation contents) of the receiving apparatus 20, the operation corresponding to each process, an operation mode of the receiving apparatus 20, which is associated with each process, and a restriction on an input operation on the receiving apparatus 20 in each operation mode. The examination flow management unit 211 manages examination progress based on such examination flow information.

The operation mode setting unit 212 sets the operation mode of the receiving apparatus 20 to the health care professional mode or patient mode based on the examination flow information. More specifically, the operation mode setting unit 212 sets the operation mode of the receiving apparatus 20 to an operation mode associated with a process in a current examination flow as well as switching the operation modes when a predetermined operation is inputted by the operation input unit 204.

The control unit 210 causes the units of the receiving apparatus 20 to execute an operation in accordance with examination progress, or restrict the execution of the operation based on the examination flow information, according to an operation mode set by the operation mode setting unit 212. For example, as illustrated in FIG. 4, it may be set such that various types of input are possible in principle while the health care professional mode is set, whereas only touch operations on images including icons and other diagrams, tables, character strings, and the like, which are displayed on a screen at a predetermined timing, can be accepted in principle while the patient mode is set. As an exception, there is an operation performed when the setting is changed from the patient mode to the health care professional mode. In this case, it may be set such that even if the patient mode is set, only an operation for displaying a password input screen and a password input operation can be accepted.

Referring to FIG. 1 again, the control apparatus 30 is realized by a workstation or personal computer including a display unit 301 such as a CRT display or liquid crystal display. The control apparatus 30 includes, for example, a USB (Universal Serial Bus) port, and is connected to the cradle 40 via the USB port. The cradle 40 is a reading device that reads image data and various information related to the examination, which are accumulated in the memory 209 of the receiving apparatus 20. When the receiving apparatus 20 is mounted on the cradle 40, the receiving apparatus 20 is electrically connected to the control apparatus 30, and image data and related information (reception strength information and time information, information related to the examination, and the like), which are stored in the memory 209, are transferred to the control apparatus 30. The control apparatus 30 performs predetermined imaging processing on the image data acquired in this manner, and causes its own display unit 301 to display in-vivo images.

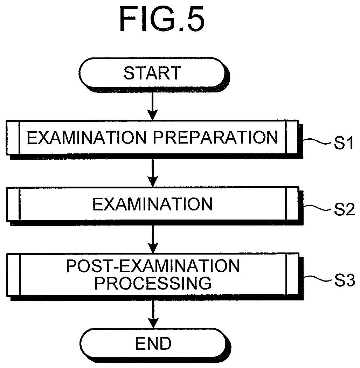

Next, a description will be given of the operations of the capsule endoscope system 1 according to the first embodiment with reference to FIG. 5. As illustrated in FIG. 5, an endoscopy in the capsule endoscope system 1 is carried out with three stages including a stage of examination preparations that the health care professional makes while operating the receiving apparatus 20 (Step S1), an examination stage where the image data of in-vivo images are acquired in a state where the patient is carrying the receiving apparatus 20 (Step S2), and a post-examination processing stage where the health care professional operates the receiving apparatus 20 again to perform end processes of the examination, and the like (Step S3).

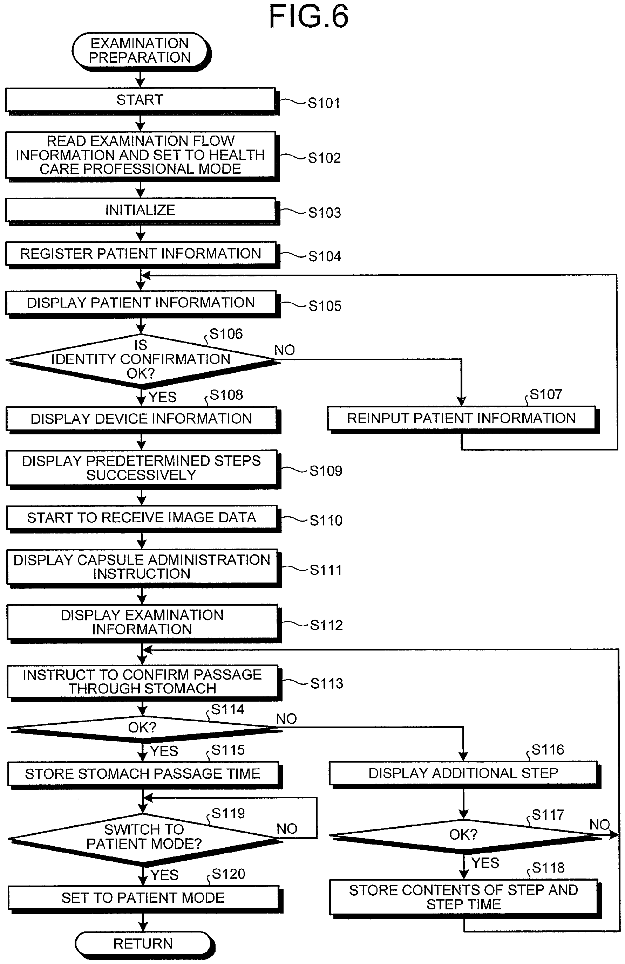

FIG. 6 is a flowchart illustrating the operations of the receiving apparatus 20 at the examination preparation stage. Part of the operations of the receiving apparatus 20, which is described below, is executed under control of the control apparatus 30 connected via the cradle 40 or a cable.

In Step S101, when the power switch 201 of the receiving apparatus 20 is long pressed, power is supplied from the battery 202 to the units of the receiving apparatus 20 to start the receiving apparatus 20.

In Step S102, the examination flow management unit 211 reads the examination flow information stored in the memory 209. Moreover, the operation mode setting unit 212 sets the operation mode of the receiving apparatus 20 to the health care professional mode.

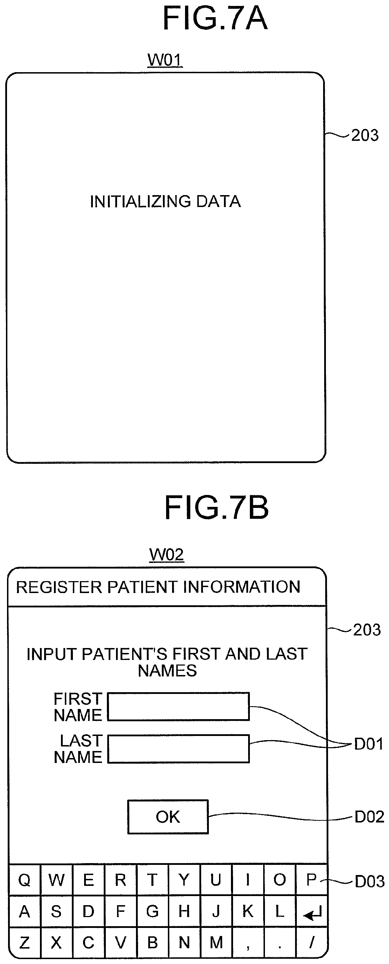

In Step S103, the receiving apparatus 20 receives an initialization instruction from the control apparatus 30, and erases (initializes) the past information stored in the memory 209. During this time, the control unit 210 may display on the display unit 203 a screen W01 on which a message to the effect that data is being initialized may be displayed, as illustrated in FIG. 7A, for example.

The initialization of the receiving apparatus 20 may be executed on the receiving apparatus 20 side in accordance with operation input by a user.

In Step S104, the receiving apparatus 20 receives patient information inputted in the control apparatus 30 to store in the memory 209, and accordingly registers the patient information. The patient information contains information such as patient name, patient ID, gender, date of birth, age, and height, weight, medical history, tastes, and contact information of the patient.

The patient information may be registered by the user directly inputting on the receiving apparatus 20 side. In this case, the control unit 210 displays on the display unit 203 a patient information registration screen W02 illustrated in FIG. 7B, for example. The screen W02 for patient information registration is a display example of a registration screen of the patient name among the patient information registration screens. The screen W02 includes a patient first name and last name input field D01, an OK icon D02, and a plurality of character keys D03 used upon text input. The control unit 210 stores the inputted patient information in the memory 209 in accordance with an input operation by touch on such a screen W02.

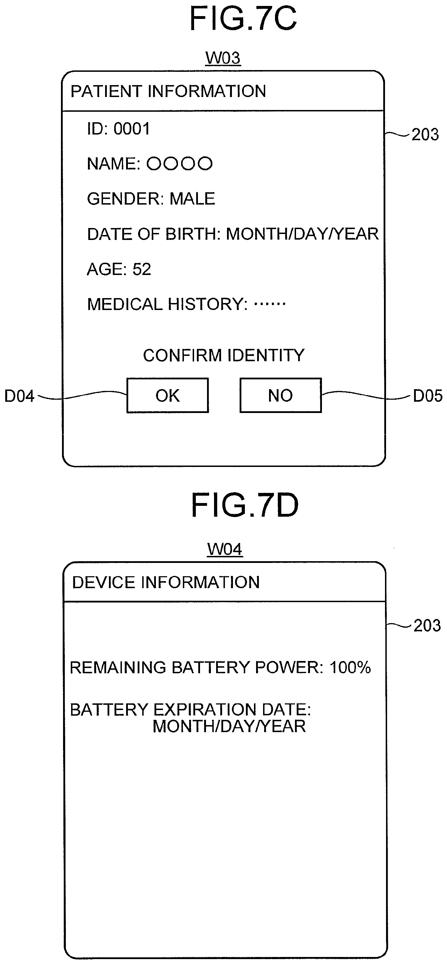

In Step S105, the control unit 210 displays the patient information stored in the memory 209 on the display unit 203 (Step S105). A screen W03 illustrated in FIG. 7C is a display example of a patient information display screen. The health care professional can confirm the identity of the patient with reference to such a screen W03. At this point, the health care professional performs an operation such as touching an OK icon D04 when the identity of the patient matches the patient information, and touching a NO icon D05 when both do not match.

The health care professional may confirm the identity of the patient while referring to the patient information displayed on the control apparatus 30.

When the OK icon D04 is selected by a touch operation on the screen W03 (Step S106: Yes), the control unit 210 shifts to the next step S108. On the other hand, when the NO icon D05 is selected on the screen W03 (Step S106: No), the control unit 210 reinputs the patient information such as by transmitting a signal to request retransmission of the patient information to the control apparatus 30 (Step S107). The operation subsequently returns to Step S104.

The initialization of the receiving apparatus 20 (step S103) and the registration of the patient information (Step S104) may be performed in advance (by the day before the examination). In this case, the receiving apparatus 20 may skip the operations of Steps S103 and S104 after startup.

In Step S108, the control unit 210 displays on the display unit 203 device information related to the receiving apparatus 20. A screen W04 illustrated in FIG. 7D is a display example of a device information display screen, and illustrates a state of displaying the remaining battery power and the battery expiration date as the device information. The health care professional can take measures such as replacement and charge of the battery as necessary with reference to such a screen W04.

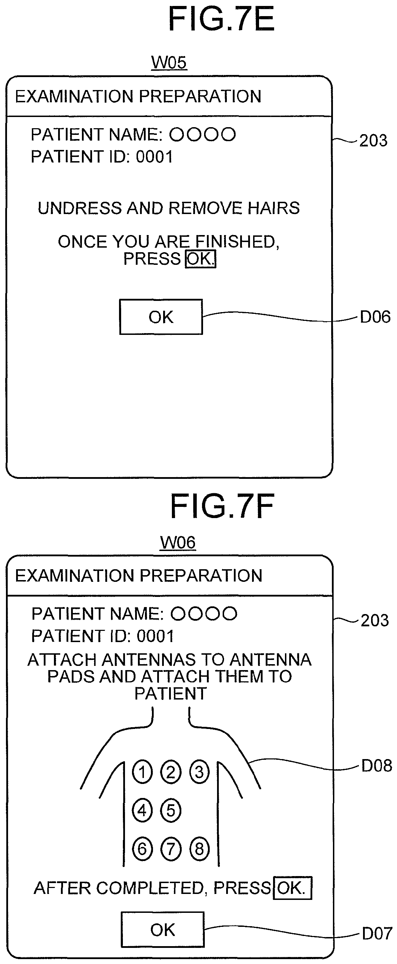

In Step S109, the control unit 210 displays on the display unit 203 predetermined steps that are necessary for the health care professional to execute for the patient before the examination in the step order. Specifically, examination preparation screens illustrated in FIGS. 7E to 7N are successively displayed.

A screen W05 illustrated in FIG. 7E is a display example of a screen to instruct the patient to execute the steps of undressing and shaving hair. The health care professional can perform specified steps on the patient on the instruction displayed on such a screen W05. In accordance with a touch operation on an OK icon D06 on the screen W05, the control unit 210 stores in the memory 209 the acceptance time of the touch as the step time of the step. Moreover, after such a confirmation operation (such as touch on the OK icon D06) by the health care professional, the control unit 210 displays the next step to be taken by the health care professional on the display unit 203.

A screen W06 illustrated in FIG. 7F is a display example of a screen to instruct the health care professional to take the step of attaching the antennas to the patient. The screen W06 includes an image D08 representing the positions to attach the antennas on the body surface of the patient. In the image D08, the positions to attach the antennas are expressed in the identification numbers (1 to 8) of the receiving antennas 21a to 21h, respectively. The health care professional can check the procedure for attaching the antennas and the appropriate positions to attach the receiving antennas 21a to 21h by referring to such an image D08. After attaching the antennas to the antenna pads on the instruction displayed on the screen W06, the health care professional attaches the antennas together with the antenna pads to the positions indicated in the image D08, and touches an OK icon D07. In accordance with the touch operation, the control unit 210 stores the acceptance time of the touch as the step time of the step in the memory 209, and displays the next step to be taken by the health care professional on the display unit 203.

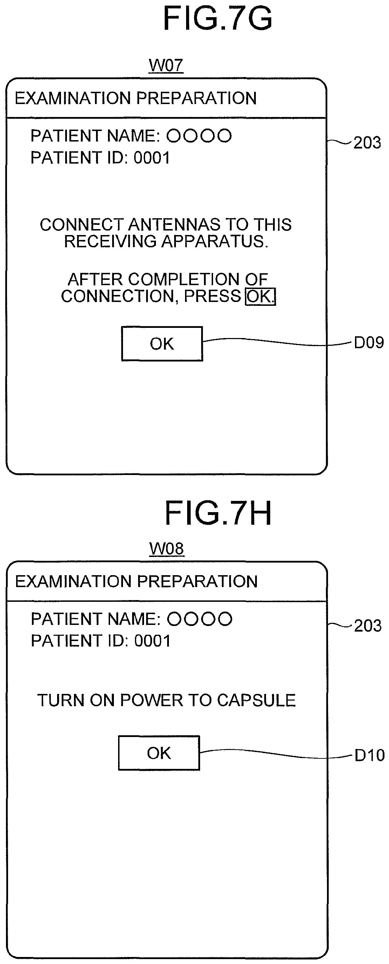

A screen W07 illustrated in FIG. 7G is a display example of a screen to instruct the health care professional to execute the step of connecting the receiving antennas 21a to 21h to the receiving apparatus 20. In addition, the control unit 210 may display on the display screen W07 the steps of handling antenna cables, attaching a pouch for fixing the receiving apparatus, and the like. The health care professional can advance complicated steps according to a specified procedure by referring to such a screen W07. In accordance with a touch operation on an OK icon D09 on the screen W07, the control unit 210 stores the acceptance time of the touch as the step time of the step (antenna connection time) in the memory 209, and displays the next step to be taken by the health care professional on the display unit 203.

A screen W08 illustrated in FIG. 7H is a display example of a screen to instruct the health care professional to turn on the power to the capsule endoscope 10. When the health care professional turns on the power to the capsule endoscope 10 on the instruction, the capsule endoscope 10 starts to capture images and wirelessly transmit the image data acquired by the capturing. In accordance with a touch operation on an OK icon D10 on the screen W08, the control unit 210 stores the acceptance time of the touch as the confirmation time of the instruction in the memory 209.

In Step S110, the control unit 210 starts to receive the image data transmitted wirelessly from the capsule endoscope 10. Moreover, the control unit 210 stores in the memory 209 the time to have started to receive the image data as the examination start time as well as measuring elapsed time from the examination start time (elapsed examination time). Furthermore, after starting to receive the image data, the control unit 210 blinks the received image display unit 205 in a predetermined color (e.g., green).

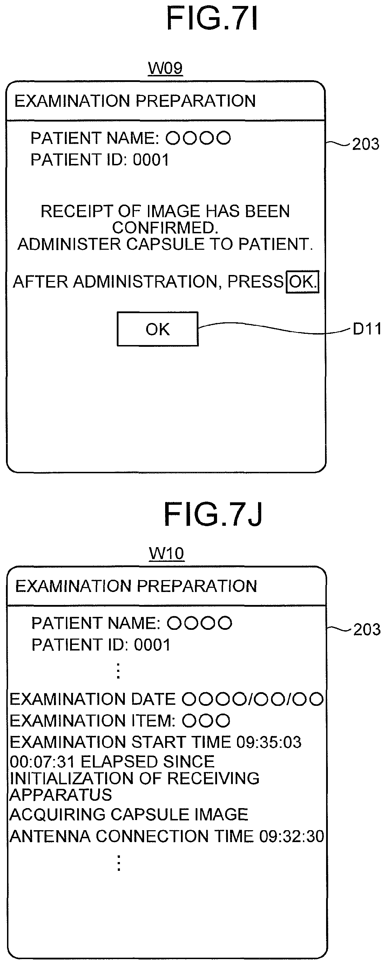

In Step S111, the control unit 210 displays on the display unit 203 a screen to instruct the health care professional to administer the capsule endoscope 10 to the patient. A screen W09 illustrated in FIG. 7I is a display example of such an instruction screen. The health care professional can grasp the timing to administer the capsule endoscope 10 to the patient by referring to the screen W09. In accordance with a touch operation on an OK icon D11 on the screen W09, the control unit 210 stores in the memory 209 the acceptance time of the touch as the administration time of the capsule endoscope 10 to the patient.

In Step S112, the control unit 210 displays patient information and examination information related to the examination, on the display unit 203. A screen W10 illustrated in FIG. 7J is a display example of the patient information and the examination information. The screen W10 includes, as the examination information, the examination date, examination items, examination start time, elapsed time from the initialization of the receiving apparatus 20, the receiving state of image data from the capsule endoscope 10, and step times such as antenna attachment. In addition, the examination information to be displayed on the display unit 203 may contain information such as the kind of a medication, and administration time of when a predetermined medication is administered.

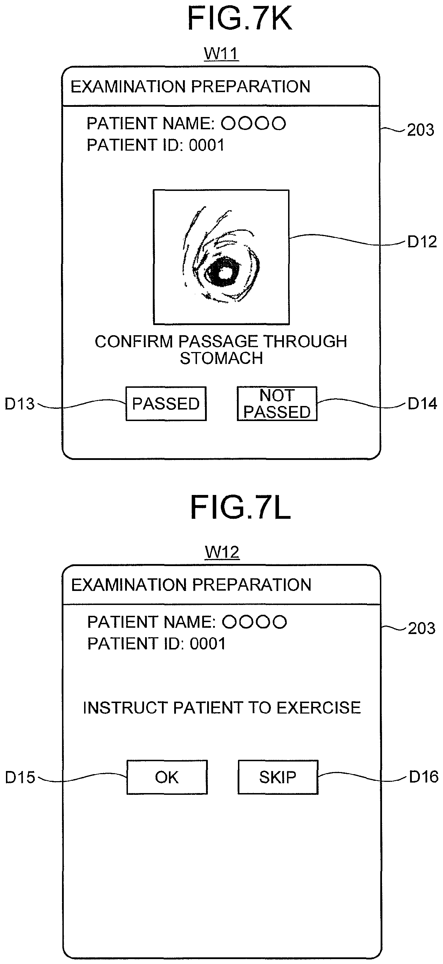

In Step S113, after a lapse of a predetermined time (e.g., approximately 30 minutes) from the examination start time, the control unit 210 displays on the display unit 203 a screen to instruct the health care professional to confirm whether or not the capsule endoscope 10 has passed through the stomach of the patient. A screen W11 illustrated in FIG. 7K is a display example of such an instruction screen. The screen W11 includes an image display area D12 to display an image based on the image data transmitted wirelessly from the capsule endoscope 10, and a passed icon D13 and a not-passed icon D14 for the health care professional inputting the confirmation result. The health care professional can determine whether or not the capsule endoscope 10 has passed through the stomach from the color and the like of an image displayed in the image display area D12.

When the passed icon D13 is selected by a touch operation on the screen W11 (Step S114: Yes), the control unit 210 stores the acceptance time of the touch as the stomach passage time in the memory 209 (Step S115).

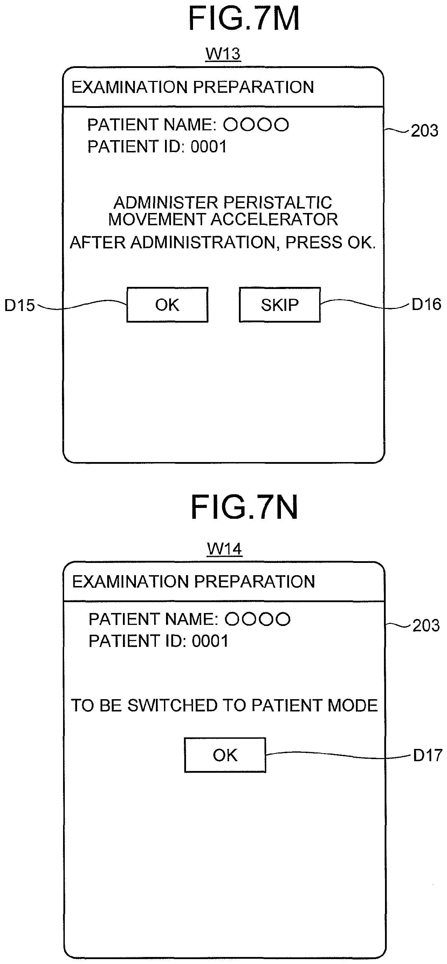

On the other hand, when the not-passed icon D14 is selected on the screen W11 (Step S114: No), the control unit 210 displays on the display unit 203 an additional step to promote the movement of the capsule endoscope 10 (Step S116). For example, a screen W12 illustrated in FIG. 7L is a display example of a screen to instruct the health care professional to have the patient exercise. Moreover, a screen W13 illustrated in FIG. 7M is a display example of a screen to instruct the health care professional to administer a peristaltic movement accelerator to the patient.

When an OK icon D15 is selected by touch operations on these screens W12 and W13 (Step S117: Yes), the control unit 210 stores the acceptance times of the touch as the step times of the steps in the memory 209 (Step S118). The operation subsequently returns to Step S113. On the other hand, when a SKIP icon D16 is selected on the screens W12 and W13 (Step S117: No), the operation returns to Step S113.

In Step S119, the control unit 210 displays on the display unit 203 a screen to have the health care professional confirm whether to switch the operation mode of the receiving apparatus 20 to the patient mode (Step S119). A screen W14 illustrated in FIG. 7N is a display example of confirmation screen of an operation mode change. In accordance with a touch operation on an OK icon D17 on the screen W14 (Step S119: Yes), the operation mode setting unit 212 sets the operation mode of the receiving apparatus 20 to the patient mode (Step S120). On the other hand, when the OK icon D17 is not touched (Step S119: No), the control unit 210 continues displaying on the display unit 203 the confirmation screen of an operation mode change, which is illustrated in FIG. 7N. Otherwise, in this case, after a lapse of a predetermined time (e.g., 15 minutes), the operation mode setting unit 212 may be forced to change the operation mode to the patient mode.

The instruction to confirm the passage through the stomach in Step S113 can be omitted. In this case, after the patient swallows the capsule endoscope 10 (after the OK icon D11 is touched on the screen W09 illustrated in FIG. 7I), the operation mode setting unit 212 may immediately switch the operation mode of the receiving apparatus 20 to the patient mode.

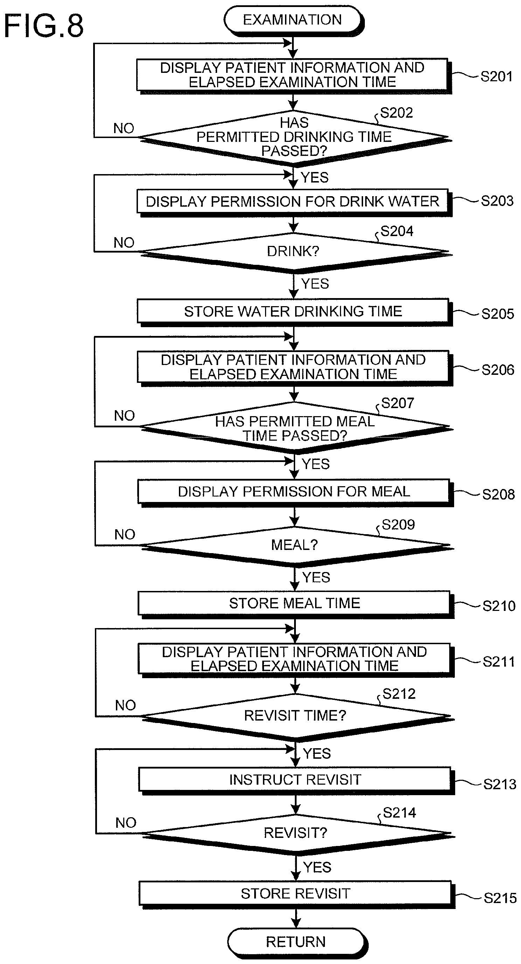

FIG. 8 is a flowchart illustrating the operations of the receiving apparatus 20 at the examination stage (Step S2 of FIG. 5). At the examination stage, the patient can freely act in a state of carrying the receiving apparatus 20.

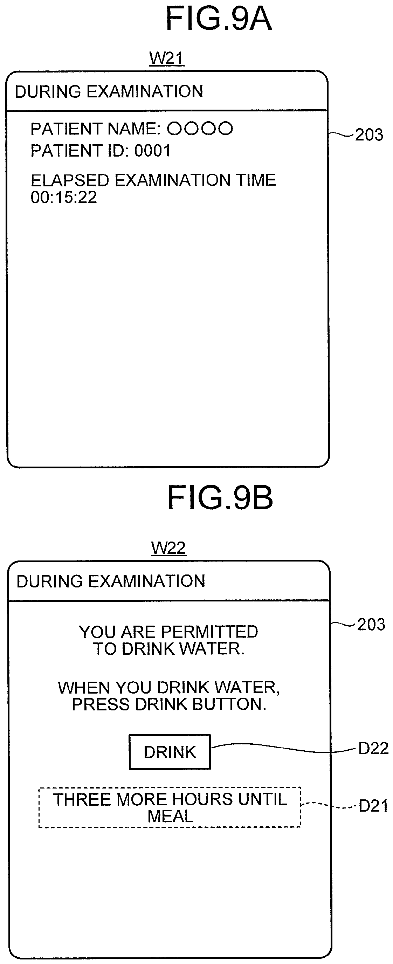

In Step S201, the control unit 210 displays the patient information and the elapsed examination time on the display unit 203. A screen W21 illustrated in FIG. 9A is a display example of the patient information and the elapsed examination time. The patient can roughly grasp the progress of the examination by referring to the elapsed examination time displayed on the display unit 203. Information displayed on a screen in patient mode is limited to the minimum information required for the patient to grasp the progress of the examination.

When a permitted water-drinking time (a predetermined time until the patient is permitted to drink water) passes from the examination start time (Step S202: Yes), the control unit 210 displays a screen to give the patient permission to drink water on the display unit 203 (Step S203). A screen W22 illustrated in FIG. 9B is a display example of a water-drinking permission screen. At this point, the control unit 210 may display an advance message D21 of a permitted future action together. Moreover, until the permitted water-drinking time passes (Step S202: No), the control unit 210 leaves the patient information and the elapsed examination time displayed on the display unit 203 (Step S201).

In accordance with a touch operation on a drink icon D22 on the screen W22 (Step S204: Yes), the control unit 210 subsequently stores the acceptance time of the touch as the water-drinking time in the memory 209 (Step S205). The control unit 210 subsequently displays the patient information and the elapsed examination time again on the display unit 203 (Step S206). On the other hand, when the drink icon D22 is not touched (Step S204: No), the operation returns to Step S203.

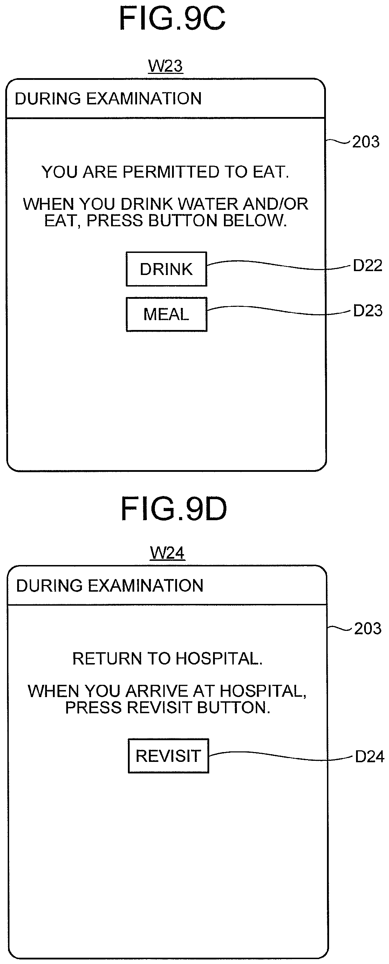

Moreover, when a permitted meal time (a predetermined time until the patient is permitted to eat) passes from the examination start time (Step S207: Yes), the control unit 210 displays a screen to permit the patient a meal on the display unit 203 (Step S208). A screen W23 illustrated in FIG. 9C is a display example of a meal permission screen. Until the permitted meal time passes (Step S207: No), the control unit 210 continues displaying the patient information and the elapsed examination time on the display unit 203 (Step S206).

In accordance with a touch operation on a meal icon D23 on the screen W23 (Step S209: Yes), the control unit 210 stores the acceptance time of the touch as the meal time in the memory 209 (Step S210). The control unit 210 subsequently displays the patient information and the elapsed examination time again on the display unit 203 (Step S211). On the other hand, when the meal icon D23 is not touched (Step S209: No), the operation returns to Step 5208.

Furthermore, when a predetermined time passes from the examination start time and the time when the patient returns to the hospital approaches (Step S212: Yes), the control unit 210 displays a screen to instruct the patient to revisit the hospital (Step S213). A screen W24 illustrated in FIG. 9D is a display example of a revisit instruction screen. Until the revisit time (Step S212: No), the control unit 210 continues displaying the patient information and the elapsed examination time on the display unit 203 (Step S211).

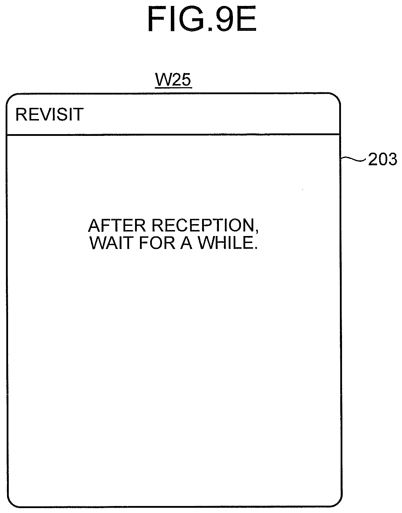

In accordance with a touch operation on a revisit icon D24 on the screen W24 (Step S214: Yes), the control unit 210 stores the acceptance time of the touch as the revisit time in the memory 209 (Step S215). The control unit 210 may subsequently display on the display unit 203 a revisit screen W25 to instruct the patient to wait, which is illustrated in FIG. 9E, for example. On the other hand, when the revisit icon D24 is not touched (Step S214: No), the operation returns to Step S213.

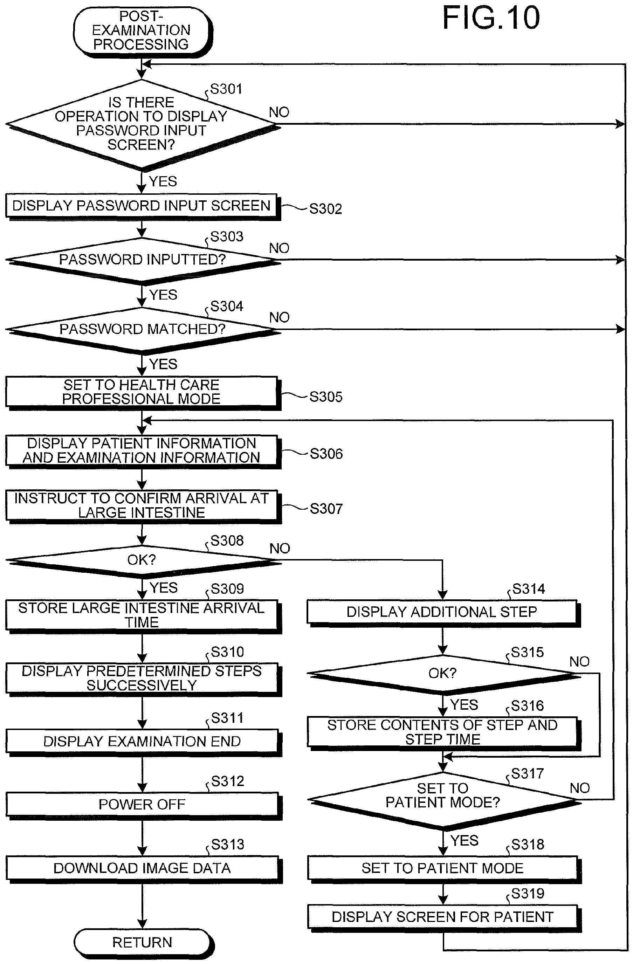

FIG. 10 is a flowchart illustrating the operations of the receiving apparatus 20 at the post-examination processing stage (Step S3 of FIG. 5). At the post-examination processing stage, the receiving apparatus 20 is assumed to be operated by the health care professional again.

In Step S301, the control unit 210 determines whether or not there has been an operation of a password input screen display for shifting to the health care professional mode, on the receiving apparatus 20. Here, the operation of the password input screen display is, for example, a predetermined operation such as touching the touch panel 204a on which the revisit screen W25 is displayed, simultaneously pressing the power switch 201 provided to the receiving apparatus 20 and the operation input unit 204 such as a push button, or successively pressing them in a predetermined order.

When there has been no operation of the password input screen display (Step S301: No), the control unit 210 waits for the operation. In this case, the control unit 210 may continue displaying on the display unit 203 the revisit screen W25 illustrated in FIG. 9E, for example.

Until the operation of the password input screen display is performed, the receiving apparatus 20 may change a screen to be displayed on the display unit 203, and the like in accordance with the action and situation of the patient. For example, while the patient is outside a facility such as a hospital, for example, the revisit screen W25 illustrated in FIG. 9E may be displayed and information related to an action in the hospital may be provided to the patient after the patient's revisit. Specifically, when recognizing a state where the patient is revisiting, the receiving apparatus 20 displays an instruction on the patient's action in the hospital (e.g., a message "please come to the examination room") on the display unit 203.

Here, the receiving apparatus 20 may recognize the patient's revisit by communication with a medical information system (not illustrated) provided in the medical institution. The medical information system that can communicate with the receiving apparatus 20 includes, for example, a medical information management server apparatus and a medical information terminal. When information indicating the patient's revisit is inputted from the medial information terminal, the medical information management server apparatus transmits the information indicating the patient's revisit to the receiving apparatus 20.

Moreover, a wireless LAN interface may be provided to the receiving apparatus 20 to perform a revisit confirmation process between the receiving apparatus 20 and the medical information management server apparatus via the wireless LAN interface. For example, upon the revisit, the receiving apparatus 20 establishes communication with the medical information management server apparatus via a wireless LAN. When the communication is established, the medical information management server apparatus regards it as the patient's revisit, and adds a flag to the effect that the patient is revisiting (hereinafter referred to as the revisit flag) to the examination data storage area in its own storage unit. When the revisit flag is added, the medical information management server apparatus notifies the receiving apparatus 20 that the patient is revisiting via the wireless LAN interface. When receiving the notification that the patient is revisiting, the receiving apparatus 20 displays information to prompt the patient's action on the display unit 203. Otherwise, a revisit mode may be provided to the receiving apparatus 20, and when receiving the revisit notification, the receiving apparatus 20 may operate in a mode to display an instruction related to the patient's action.

Moreover, the medical information management server apparatus transmits the examination information to which the revisit flag is added to the control apparatus or a terminal device carried by the health care professional. This may serve as a trigger to have the health care professional recognize the patient's revisit and prepare the examination end processes.

Moreover, when such a medical information system is provided, when detecting that the patient has entered the examination room, the receiving apparatus 20 may shift to the password input screen display.

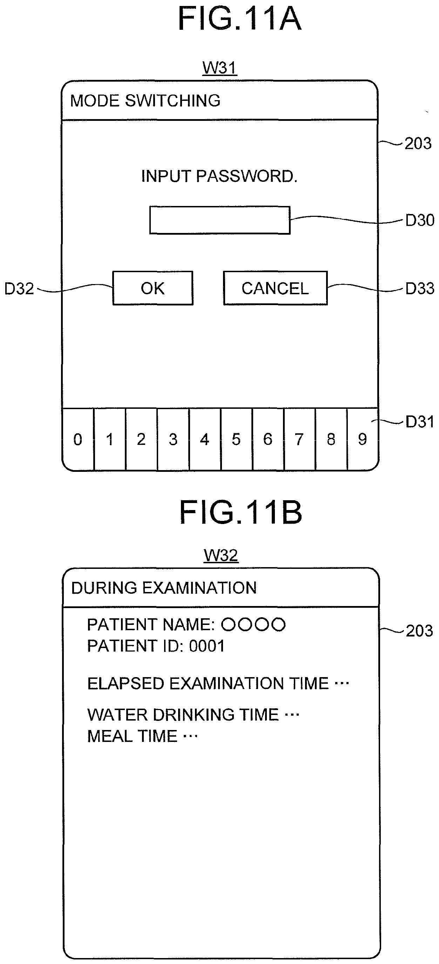

On the other hand, when the password input screen display is operated (Step S301: Yes), the control unit 210 displays on the display unit 203 a password input screen illustrated in FIG. 11A, for example (Step S302). A screen W31 illustrated in FIG. 11A is a display example of the password input screen. The screen W31 includes a password input area D30, numeric input keys D31, an OK icon D32, and a cancel icon D33. In FIG. 11A, a plurality of numeric input keys D31 is displayed; however, it may be set such that a plurality of input keys for inputting alphabets and the like is displayed. Moreover, the health care professional can erase a password that has once been inputted in the password input area D30 from the screen by a touch operation on the cancel icon D33.

When a password is inputted in the password input area D30 by a touch operation on the screen W31 (Step S303: Yes), the control unit 210 determines whether or not the inputted password matches a preset password in accordance with a touch operation on the OK icon D32 (Step S304). When the passwords match (Step S304: Yes), the operation mode setting unit 212 sets the operation mode of the receiving apparatus 20 to the health care professional mode (Step S305).

On the other hand, when a password is not inputted for a predetermined time or more (Step S303: No), or when the inputted password does not match a preset password (Step S304: No), the operation returns to Step S301.

In Step S306, the control unit 210 displays the patient information and the examination information on the display unit 203. A screen W32 illustrated in FIG. 11B is a display example of the patient information and the examination information. The examination information displayed on this screen contains the action record information of the patient during the examination (free actions), such as a water-drinking time and a meal time, which is acquired during the examination, in addition to the examination information displayed in Step S112. The health care professional can confirm the identity of the patient and check the actions of the patient during free actions by referring to such a screen W32.

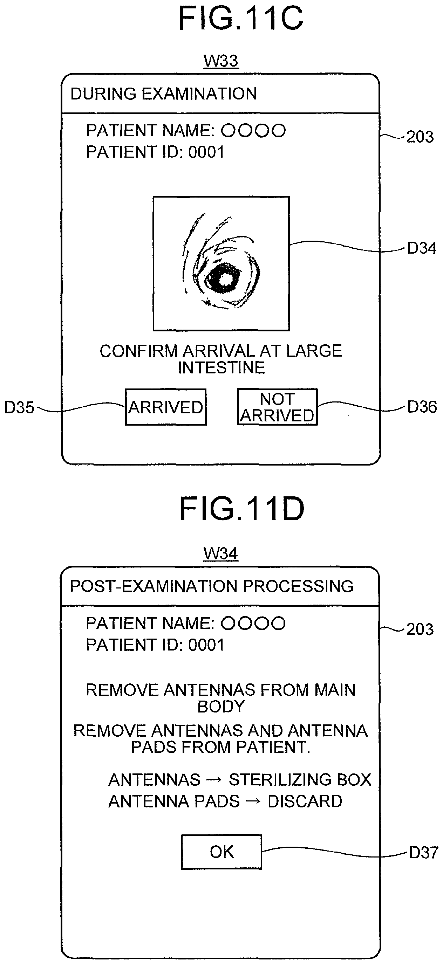

After a lapse of a predetermined time (e.g., approximately 6 hours) from the examination start time, the control unit 210 displays on the display unit 203 a screen to instruct the health care professional to confirm whether or not the capsule endoscope 10 has arrived at the large intestine of the patient (Step S307). A screen W33 illustrated in FIG. 11C illustrates a display example of such an instruction screen. The screen W33 includes an image display area D34 to display images based on image data transmitted wirelessly from the capsule endoscope 10, and an arrived icon D35 and a not-arrived icon D36 for the health care professional inputting the confirmation result. The health care professional can determine whether or not the capsule endoscope 10 has arrived at the large intestine from the color or the like of the image displayed on the image display area D34.

When the arrived icon D35 is selected by a touch operation on the screen W33 (Step S308: Yes), the control unit 210 stores the acceptance time of the touch as the large intestine arrival time in the memory 209 (Step S309).

In Step S310, the control unit 210 displays on the display unit 203 predetermined steps to be executed by the health care professional after the examination in the step order. A screen W34 illustrated in FIG. 11D is a display example of steps to be executed by the health care professional. In accordance with a touch operation on an OK icon D37 on the screen W34, the control unit 210 stores the acceptance time of the touch as the examination end time in the memory 209.

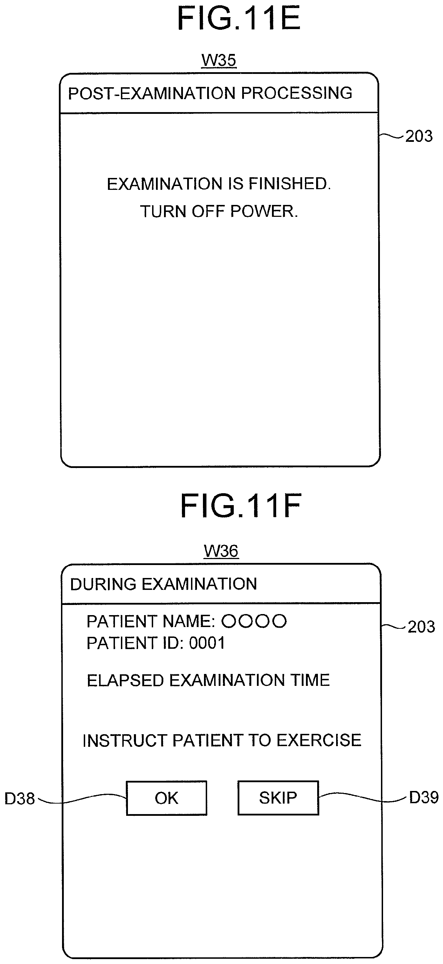

In Step S311, the control unit 210 displays an examination end screen on the display unit 203. A screen W35 illustrated in FIG. 11E is a display example of the examination end screen. The receiving apparatus 20 subsequently turns off the power in accordance with a predetermined end operation (e.g., a long press on the power switch 201) (Step S312). The receiving apparatus 20 may turn off the power automatically when a predetermined time passes after the display of the examination end screen.

In Step S313, when the receiving apparatus 20 is mounted on the cradle 40 (refer to FIG. 1), information stored during the time from the examination preparation to the post-examination processing, in addition to the image data and the related information, which are stored in the memory 209, is downloaded to the control apparatus 30. The control apparatus 30 stores these image data and information in its own built-in memory as well as executing predetermined processes. Specifically, the control apparatus 30 generates a series of in-vivo images by performing predetermined image processing on the image data, and generates an interpretation screen where these in-vivo images are arranged in a predetermined format, or creates an interpretation report.

On the other hand, when the not-arrived icon D36 is selected on the screen W33 (Step S308: No), the control unit 210 displays on the display unit 203 an additional step to promote the movement of the capsule endoscope 10 (Step S314). For example, FIG. 11F is a display example of a screen to instruct the health care professional to have the patient exercise.

When an OK icon D38 is selected by a touch operation on the screen W36 (Step S315: Yes), the control unit 210 stores the acceptance time of the touch as the step time of the step in the memory 209 (Step S316). On the other hand, when a SKIP icon D39 is selected on the screen W34 (Step S315: No), the operation shifts directly to Step S317.

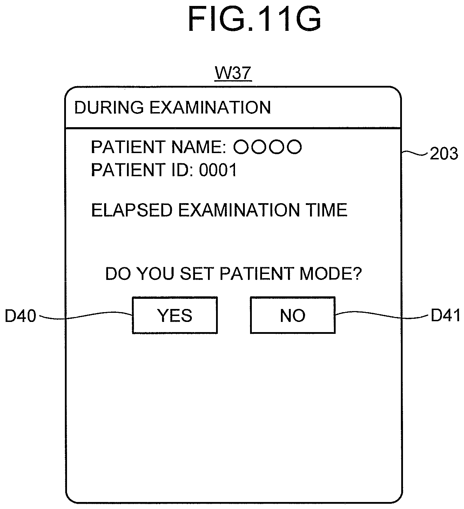

In Step S317, the control unit 210 displays on the display unit 203 a screen to check with the health care professional whether to switch the operation mode of the receiving apparatus 20 to the patient mode again. A screen W37 illustrates in FIG. 11G is a display example of check screen of an operation mode change.

When a setting instruction to the patient mode is inputted by a touch operation on an Yes icon D40 on the screen W37 (Step S317: Yes), the control unit 210, the operation mode setting unit 212 sets the operation mode of the receiving apparatus 20 to the patient mode again (Step S318). In this case, the health care professional can attach the receiving apparatus 20 to the patient again, and give some further free time to the patient.

In Step S319, the control unit 210 displays on the display unit 203 screens for the patient (screens to be displayed in the patient mode, such as the screen W21 including the patient information and the elapsed examination time, which is illustrated in FIG. 9A, and the screen W25 to instruct the patient to wait, which is illustrated in FIG. 9E). At this point, after the setting of a predetermined time and a lapse of the predetermined time, the control unit 210 may display action instructions (such as an instruction to check in again, and an instruction to contact the health care professional) on the screen for the patient on the display unit 203.

The operation subsequently returns to Step S301.

On the other hand, when the setting instruction to the patient mode is not inputted due to a touch operation on a NO icon D41 on the screen W37 (Step S317: No), the operation returns to Step S306.

As described above, according to the first embodiment, the operation mode of the receiving apparatus 20 is switched between the health care professional mode and the patient mode in accordance with the progress of the examination flow; accordingly, it is possible to suppress the occurrence of an operating error even under the circumstance where the health care professional and the patient use the common receiving apparatus 20. Especially, the patient mode where the functions and input operations are restricted is set during the examination during which the patient can act freely. Accordingly, it is possible to prevent a failure in the examination due to an operating error, and the like.

Moreover, according to the first embodiment, a switch from the patient mode to the health care professional mode requires the input of a password; accordingly, it is possible to prevent a situation where the setting is accidentally changed by the patient's operation to the health care professional mode.

Moreover, according to the first embodiment, operations to be executed by the health care professional and steps for the patient are successively displayed on the display unit 203 of the receiving apparatus 20 while the health care professional mode is set; accordingly, the health care professional can surely implement necessary steps without referring to a manual and the like separately.

For example, the attachment positions of the receiving antennas 21a to 21h to be attached to the patient are different according to the manufacturers; accordingly, confusion may arise at a facility such as a hospital that handles apparatuses of a plurality of manufacturers. However, according to the first embodiment, it is possible to display the attachment positions of the receiving antennas 21a to 21h on an image on a manufacturer basis; accordingly, it becomes possible for the health care professional to surely attach the receiving antennas 21a to 21h at appropriate positions by referring to the displayed image. Therefore, it is possible to avoid false detection of the position of the capsule endoscope 10 due to a mistake in the attachment positions of the receiving antennas 21a to 21h, and the like.

Moreover, according to the first embodiment, while the health care professional mode is set, an instruction on the next step is displayed on the display unit 203 after the implementation of a certain step is confirmed (such as touch on an OK icon); accordingly, it becomes possible even for a health care professional who has little experience on the implementation of a capsule endoscopy to implement steps without making a mistake in the procedure.

Moreover, according to the first embodiment, the contents of work implemented by the health care professional, the time required for the work, and the like are stored in the memory 209; accordingly, it is possible to collect these pieces of information to utilize as basic data for promoting working efficiency.

Moreover, according to the first embodiment, action permission and a necessary action instruction to the patient are displayed on the display unit 203 of the receiving apparatus 20 during the patient's free actions; accordingly, the patient can take an appropriate action even while away from the hospital. Furthermore, actions taken by the patient during the examination (action record) are stored in the memory 209; accordingly, it is possible to utilize the action record as reference information upon diagnosis of an in-vivo image related to the patient.

### (Modification 1-1)

In the first embodiment, the operation input unit 204 is realized by the touch panel 204a; however, the operation input unit 204 may be any configuration. For example, input devices such as a keyboard, a wheel, and a push button may be provided to the receiving apparatus 20, instead of or in addition to the touch panel 204a.

Moreover, the receiving apparatus 20 may be provided with a barcode reader. In this case, the patient information can be inputted into the receiving apparatus 20 by causing the barcode reader to read a barcode described in the patient's chart or the like, or the password can be inputted into the receiving apparatus 20 by causing the barcode reader to read a barcode described on a name tag of the health care professional, or the like.

### (Modification 1-2)

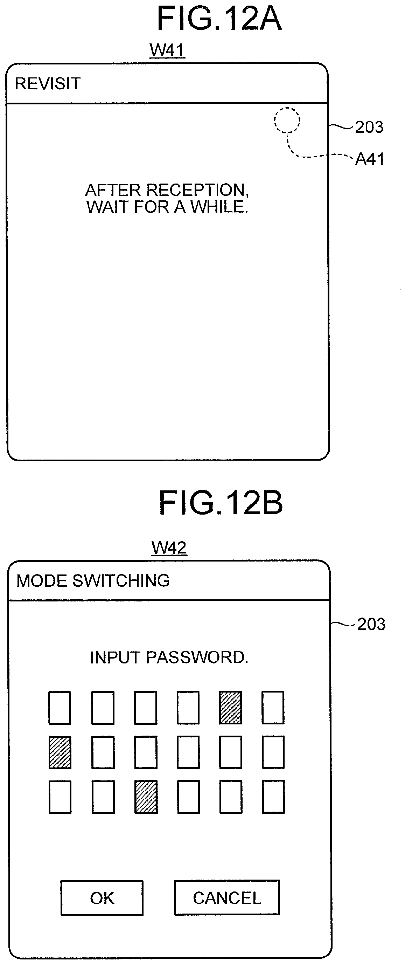

As the operations of the password input screen display (Step S301), various operations can be set in addition to the operations set in the first embodiment. For example, the operations may include not only simply touching the touch panel 204a, but also the operation of touching the touch panel 204a in a predetermined pattern (e.g., a predetermined number of touch at predetermined time intervals), the operation of touching a predetermined area A41 on a revisit screen W41 as illustrated in FIG. 12A, or any combination thereof. At this point, in the area A41, an icon that the health care professional can recognize may be displayed, or nothing is displayed to prevent an operating error by the patient.

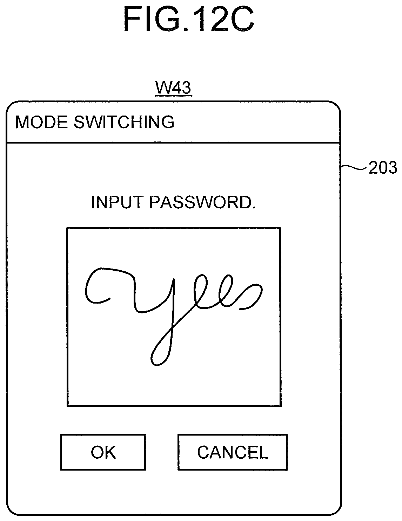

Moreover, the password input system is also not limited to the system described in the first embodiment. For example, as illustrated in FIG. 12B, a system where a preregistered pattern is touched on the screen W42 is also acceptable. Otherwise, as illustrated in FIG. 12C, a system where a preregistered signature is written as a locus on the screen W43 is also acceptable.

A password may be assigned to each health care professional, or assigned for every examination or whenever the patient goes out. In the former case, a password inputted upon change of the setting from the patient mode to the health care professional is stored in the memory 209; accordingly, it later becomes possible to extract the health care professional being a holder of the password and make work responsibility clear. On the other hand, in the latter case, it becomes possible to reduce the risk of occurrence of trouble due to the leak of the password.

Moreover, in the first embodiment, when the setting of the operation mode of the receiving apparatus 20 is changed from the patient mode to the health care professional mode, the health care professional is requested to input a password. However, a password may be requested to be inputted on startup of the receiving apparatus 20. In this case, a password is set for each health care professional; accordingly, it is possible to identify the executer of subsequent processes.

### (Modification 1-3)

An image display device may be connected to the receiving apparatus 20 separately to display an image and the like, which are displayed on the display unit 203, on the image display device. In this case, the health care professional can confirm passage through the stomach, arrival at the large intestine, and the like while observing in-vivo images displayed on the image display device.

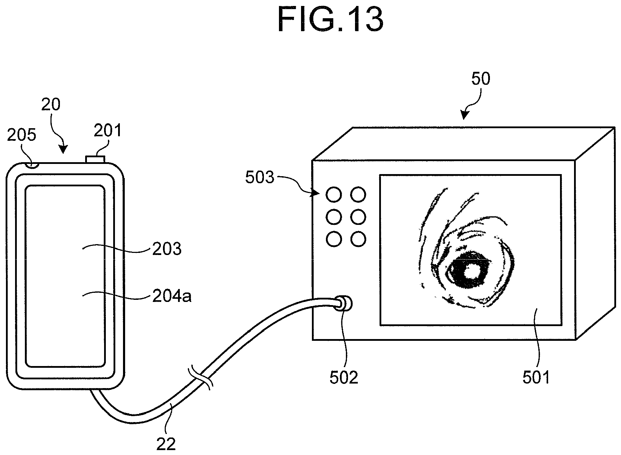

FIG. 13 is a schematic diagram illustrating an example where the receiving apparatus 20 is connected to a real time viewer 50 that visualizes image data received by the receiving apparatus 20 from the capsule endoscope 10, in real time. The real time viewer 50 includes a display unit 501 realized by a display panel such as a liquid crystal or organic EL, and a connecting terminal 502 to be connected to the receiving apparatus 20 via a cable 22, and an operation input unit 503 such as an adjustment knob.

According to Modification 1-3, in-vivo images are displayed on the real time viewer 50 that can execute further image processing on the image data; accordingly, the health care professional can confirm passage through the stomach, arrival at the large intestine, and the like more accurately.

### (Modification 1-4)

In the first embodiment, the receiving apparatus 20 is set to the patient mode to make it impossible to accept the input of an operation on the receiving apparatus 20 during the examination. However, the receiving apparatus 20 may change the setting to the health care professional mode at any time even during the examination to make it possible for the health care professional to observe in-vivo images and execute various processes. In this case, the receiving apparatus 20 may be configured such that the password input screen is displayed at any time on the display unit 203 in accordance with the operation input described in Modification 1-1, for example.

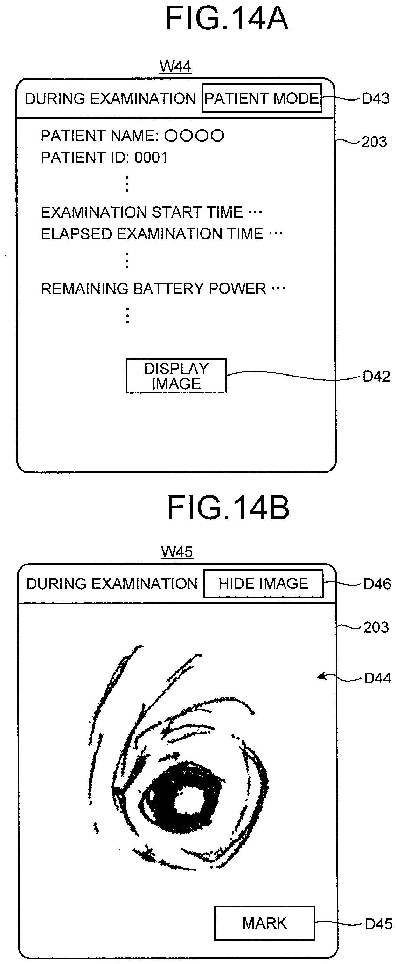

FIG. 14A is an example of a screen to be displayed when the receiving apparatus 20 changes the setting to the health care professional mode during the examination. Further detailed information (patient information, examination information, device information, and the like) than the case of the patient mode is displayed on a screen W44 illustrated in FIG. 14A. For example, as illustrated in FIG. 14B, in accordance with a touch operation on a display-image icon D42 on the screen W44, the control unit 210 displays on the display unit 203 a screen W45 all over which an in-vivo image D44 is displayed. The health care professional can grasp the situation of the examination by observing such a screen W45.

Moreover, in accordance with a touch operation on a mark icon D45 on the screen W45, the control unit 210 stores in the memory 209 image data associated with an in-vivo image displayed at the time while adding marking information to the image data. In this manner, the in-vivo image to which the marking information is added can be extracted after the image data is downloaded into the control apparatus 30.

Furthermore, in accordance with a touch operation on a hide-image icon D46 on the screen W45, the control unit 210 causes the display of the display unit 203 to transit to the original screen W44.

Moreover, in accordance with a touch operation on a patient mode icon D43 on the screen W44, the operation mode setting unit 212 sets the operation mode of the receiving apparatus 20 to the patient mode again.

According to Modification 1-4, the health care professional can mark an in-vivo image of interest while observing in-vivo images during the examination in real time; accordingly, it becomes possible to improve efficiency in a subsequent interpretation work. Moreover, the operation mode of the receiving apparatus 20 can be switched any time in accordance with the operation of the health care professional; accordingly, in relation to the receiving apparatus 20, it enables the health care professional to input an operation at an arbitrary timing and is possible to prevent an operating error by the patient.

### (Modification 1-5)

In the first embodiment, image data accumulated in the memory 209 of the receiving apparatus 20 are downloaded into the control apparatus 30 using the cradle 40. However, the downloading system is not limited to the system where the receiving apparatus 20 communicates directly with the control apparatus 30 via the cradle 40.

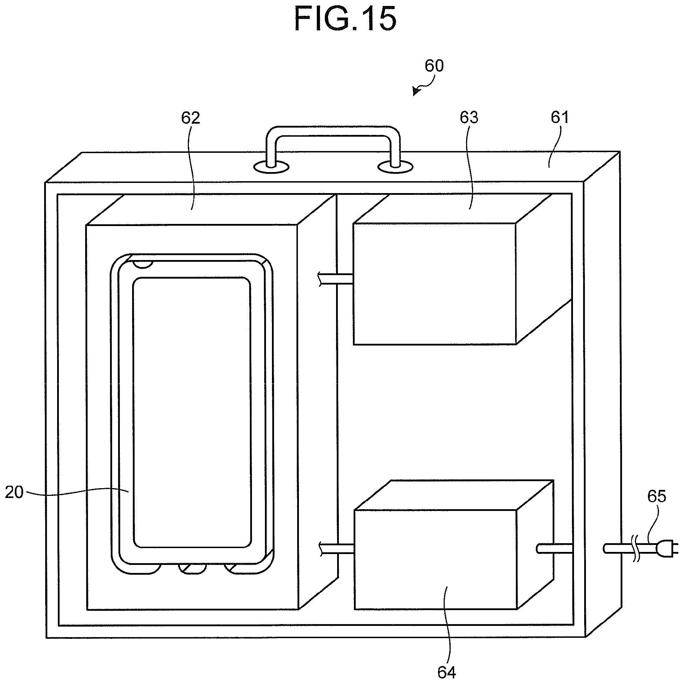

FIG. 15 is a schematic diagram illustrating a configuration example of a storage case type of download device. A download device 60 illustrated in FIG. 15 includes a casing 61 being a storage case of the receiving apparatus 20, a holder 62 for the receiving apparatus 20, the holder being stored in the casing 61, a communication unit 63 that performs wired or wireless communication with the control apparatus 30, and a rechargeable battery unit 64. The holder 62 holds the receiving apparatus 20 as well as electrically connecting the receiving apparatus 20 to the communication unit 63 and the battery unit 64, respectively, via itself. Power is supplied from the battery unit 64 to the communication unit 63 via the holder 62 and the receiving apparatus 20. The battery unit 64 is connected to a power code 65 for receiving power from the outside.

When the receiving apparatus 20 is set on the holder 62 after the end of the examination, the communication unit 63 successively transmits the image data accumulated in the memory 209 by wire or wirelessly to the control apparatus 30. When the transfer of the image data ends, then the battery unit 64 charges the receiving apparatus 20. The receiving apparatus 20 continues to be stored in the casing 61.

According to such the download device 60, it is possible to download the image data and put the receiving apparatus 20 away at the same time. Therefore, it becomes possible to dramatically shorten the health care professional's working time that conventionally requires two stages of downloading and putting the receiving apparatus 20 away.

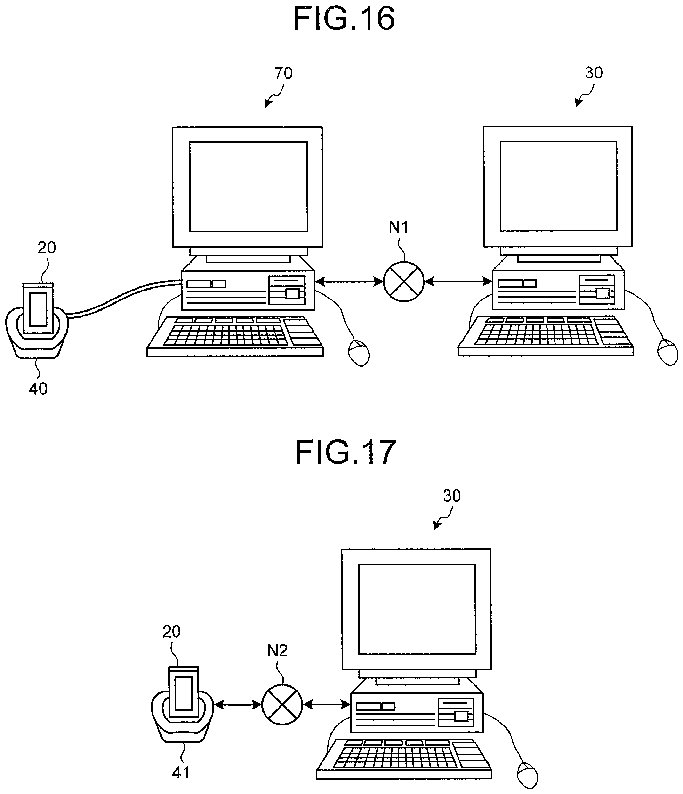

FIG. 16 is a schematic diagram illustrating a configuration example where the image data accumulated in the memory 209 of the receiving apparatus 20 are downloaded into a computer 70 being different from the control apparatus 30, which is provided in a manner of being able to communicate with the control apparatus 30. The computer 70 is, for example, a personal computer installed in a hospital, or a private personal computer of the health care professional. In the configuration example, the cradle 40 is connected to the computer 70. When the receiving apparatus 20 is mounted on the cradle 40 after the end of the examination, the image data accumulated in the memory 209 of the receiving apparatus 20 are transferred to the computer 70 successively. The image data downloaded into the computer 70 are transmitted at an arbitrary timing to the control apparatus 30 via a communication network N1 connected by wire or wirelessly.

According to the configuration example, it is possible to immediately start downloading the image data from the receiving apparatus 20 even under circumstances such as that the cradle 40 connected to the control apparatus 30 is in use or the image data cannot be immediately transferred to the control apparatus 30 for reasons such as that the examination was carried out at a remote place from the control apparatus 30. Therefore, it is possible to advance work such as putting the receiving apparatus 20 away without waiting time, which makes it possible to improve efficiency in the work of the health care professional. Moreover, according to the configuration example, it becomes possible to download image data into a general-purpose personal computer; accordingly, it becomes possible to improve the convenience of the receiving apparatus and the cradle.

FIG. 17 is a schematic diagram illustrating a configuration example where the image data accumulated in the memory 209 of the receiving apparatus 20 are downloaded using a cradle having a communication function. A cradle 41 illustrated in FIG. 17 has a wired or wireless communication function in addition to the normal download function. When the receiving apparatus 20 is mounted on the cradle 41 after the end of the examination, the image data accumulated in the memory of the receiving apparatus 20 are transferred successively to the control apparatus 30 via a communication network N2 to which the cradle 41 is connected by wire or wirelessly.

According to the configuration example, there is no need that the installation place of the control apparatus 30 is necessarily brought near to the implementation place of downloading. Therefore, a degree of flexibility in layout increases such as that the control apparatus 30 that is mainly operated by a doctor is installed in a consultation room or reading room, and the receiving apparatus 20 and the cradle 41, which are mainly operated by a nurse, are placed in the examination room.

### (Modification 1-6)

In the first embodiment, the examination flow management unit 211 controls the flow of processes from the examination preparation to the post-examination processing, but may further control the flow of processes in a pre-examination schedule of the patient. In this case, the examination flow management unit 211 controls using examination flow information that information related to processes and actions to be executed by the patient before the examination (until the patient visits on the day of the examination) is further added to the examination flow information illustrated in FIG. 4. The processes and actions to be executed by the patient before the examination include the intake of a predetermined pretreatment drug, the input of a final check on the last meal before the examination, check-in, and the like. Such a pre-examination schedule is used by modifying a preset form (adding, changing, and deleting items, changing the time, and the like) in accordance with the patient (the age, constitutional predisposition, and the like of the patient) or the examination (the contents, start time, and the like of the examination).

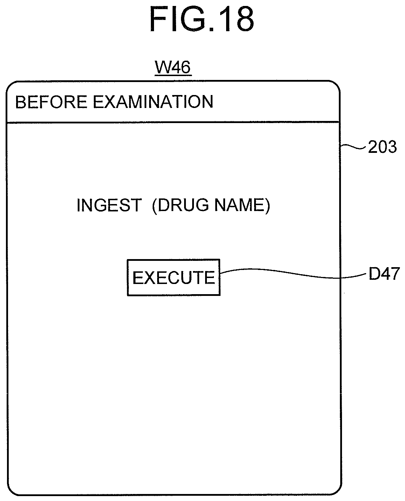

When the pre-examination schedule of the patient is managed using the receiving apparatus 20, when the patient visits a hospital to make an appointment for the examination, the receiving apparatus 20 set to the patient mode is lent to the patient. The receiving apparatus 20 displays a message to the patient on the display unit 203 when the time when the patient needs to execute a predetermined process comes. For example, a screen W46 illustrated in FIG. 18 is a display example of a screen to instruct the patient to take a predetermined pretreatment drug. At this point, the receiving apparatus 20 may draw the patient's attention, such as by generating sound and vibration, or blinking the received image display unit 205 in a predetermined color (e.g., yellow). In accordance with a touch operation on an execute icon D47 on the screen W46, the control unit 210 stores the action record of the patient in the memory 209, setting the acceptance time of the touch as the time when the process is executed. The action record is transferred to the control apparatus 30 together with the image data after the end of the examination. The receiving apparatus 20 changes the setting to the health care professional mode by the input of a password by the health care professional after the patient visits a hospital on the day of the examination.

### (Modification 1-7)

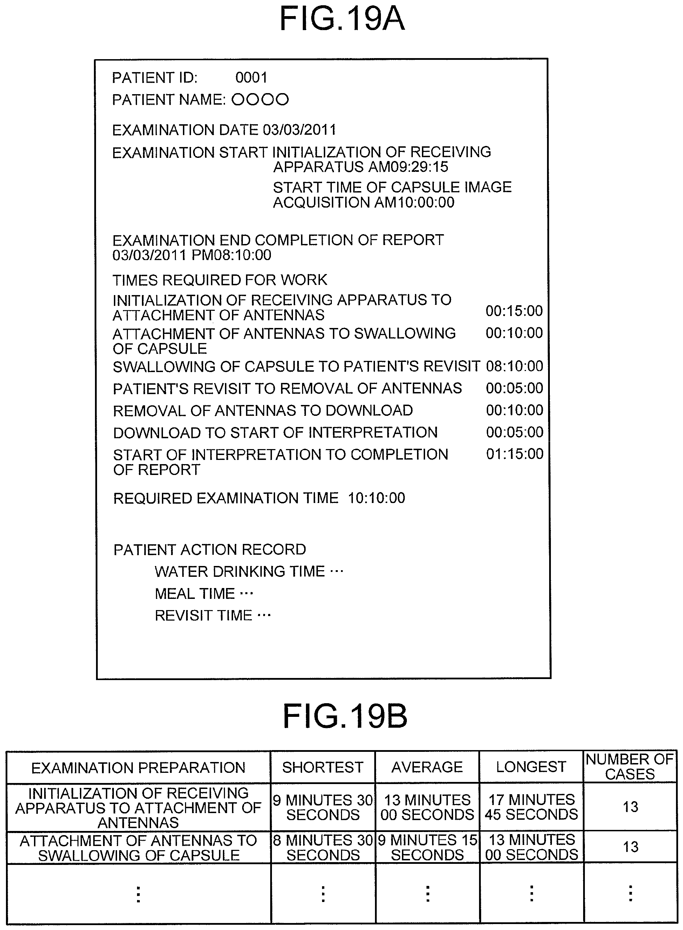

The control apparatus 30 may create an examination report based on the examination information and the patient's action record, which are stored in the receiving apparatus 20 and downloaded together with the image data. FIG. 19A is a display example of an examination report created by the control apparatus 30. Described in the examination report illustrated in FIG. 19A is information such as the contents of processes (an operation on the receiving apparatus 20, a step for the patient, and the like) to have been executed by the health care professional, the times to have executed the processes, the time required for the respective processes, and the patient's actions (contents and times) during the examination. The health care professional can use such an examination report as reference information and the like upon medical diagnosis of the patient.

Moreover, the control apparatus 30 may process the examination information and the patient's action record statistically. FIG. 19B is a display example of a result that the examination information is processed statistically. As illustrated in FIG. 19B, the calculation of the average value, the shortest time, the longest time, and the like of the time required for the respective processes in the examination preparation, the summation of the number of cases, and the like are performed as the statistical processes. The statistical information obtained in this manner can be used as reference information upon reviewing the examination flow.

### (Second Embodiment)

Next, a description will be given of a second embodiment of the present invention.

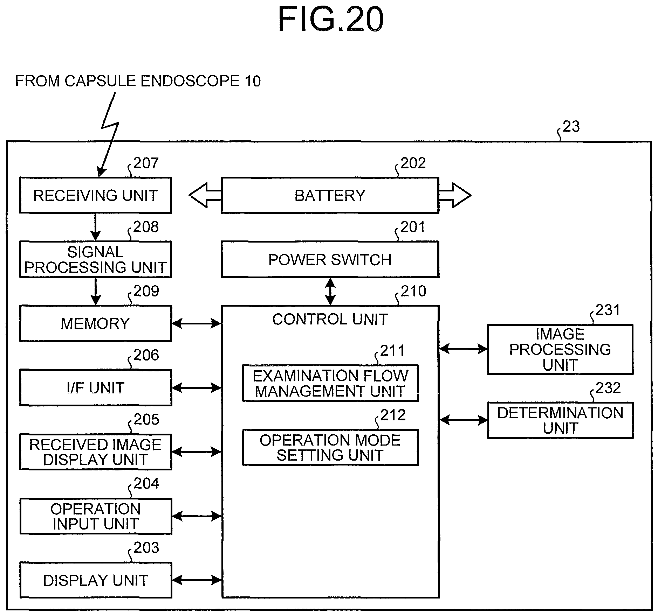

FIG. 20 is a block diagram illustrating the configuration of a receiving apparatus according to the second embodiment. The second embodiment is characterized in that the receiving apparatus automatically determines on confirmation of passage through the stomach and arrival at the large intestine of the capsule endoscope 10. The entire configuration of a capsule endoscope system in the second embodiment is similar to the one illustrated in FIG. 1.

As illustrated in FIG. 20, a receiving apparatus 23 according to the second embodiment further includes an image processing unit 231 that performs predetermined image processing on an in-vivo image associated with an image datum accumulated in the memory 209 and calculates its feature data, and a determination unit 232 that determines the situation of the in-vivo image based on the calculated feature data, in addition to the configuration of the receiving apparatus 20 (FIG. 3). The configurations and operations of the other units are similar to those illustrated in FIG. 3.

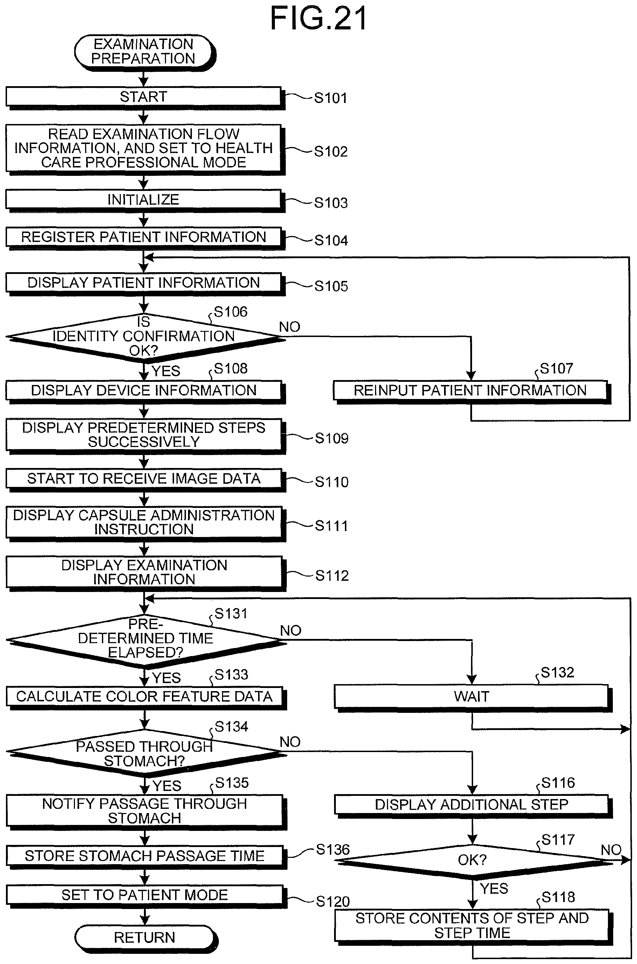

FIG. 21 is a flowchart illustrating the operations of the receiving apparatus 23 at the examination preparation stage. Steps S101 to S112, S120, and S116 to S118 illustrated in FIG. 21 correspond to those described in the first embodiment.

In Step S131 subsequent to Step S112, the control unit 210 determines whether or not a predetermined time has passed since the capsule endoscope 10 was administered to the patient. When the predetermined time has passed (Step S131: Yes), the operation shifts to Step S133. On the other hand, when the predetermined time has not passed (Step S131: No), the control unit 210 waits until the predetermined time passes (Step S132).

In Step S133, the image processing unit 231 calculates the feature data of in-vivo images based on the image data received from the capsule endoscope 10. In the second embodiment, color feature data is calculated as the feature data of the in-vivo image.

In Step S134, the determination unit 232 determines whether or not the capsule endoscope 10 has moved from the stomach to small intestine of the patient (in other words, passed through the stomach) by comparing the calculated feature data with a predetermined threshold value. For example, when it is determined from the calculated color feature data that the color of an in-vivo image has turned from yellow to red, the determination unit 232 determines that the capsule endoscope 10 has moved to the small intestine.

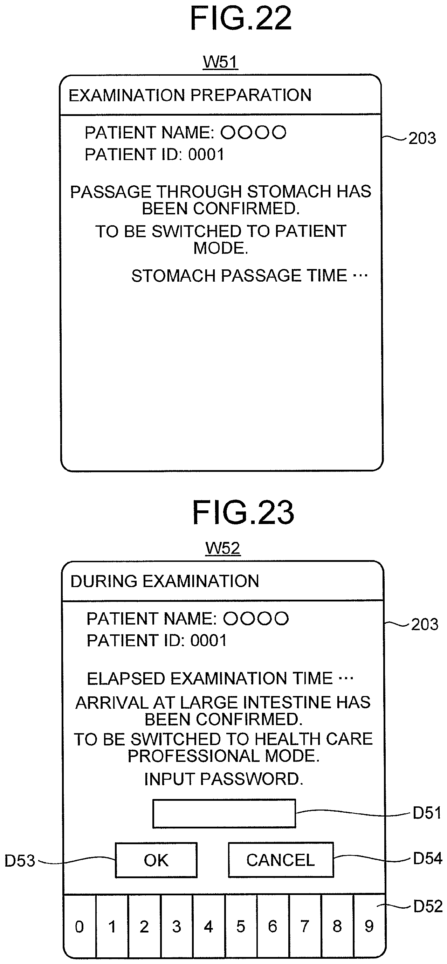

When it is determined that the capsule endoscope 10 has passed through the stomach (Step S134: Yes), the control unit 210 displays on the display unit 203 a message to notify the passage of the capsule endoscope 10 through the stomach in accordance with the determination of the determination unit 232 (Step S135). A screen W51 illustrated in FIG. 22 is a display example of such a message. Moreover, at this point, the control unit 210 stores the time when passage through the stomach is determined as the stomach passage time in the memory 209 (Step S136). The operation mode setting unit 212 subsequently switches the operation mode of the receiving apparatus 23 to the patient mode (Step S120). On the other hand, when it is determined that the capsule endoscope 10 has not passed through the stomach (Step S134: No), the operation shifts to Step S116.

Moreover, when arrival at the large intestine is confirmed at the post-examination processing stage, instead of Steps S307 and S308 illustrated in FIG. 10, the image processing unit 231 calculates the feature data (e.g., color feature data) of in-vivo images based on the image data transmitted from the capsule endoscope 10, and the determination unit 232 compares the calculated feature data with a predetermined threshold value. Accordingly, it is determined whether or not the capsule endoscope 10 has arrived at the large intestine of the patient. For example, when it is determined from the calculated color feature data that the color of an in-vivo image has turned from yellow to reddish brown, the determination unit 232 determines that the capsule endoscope 10 has arrived at the large intestine. As a result of the determination, when the capsule endoscope 10 has arrived at the large intestine, the operation shifts to Step S309. On the other hand, when not arrived at the large intestine, the operation shifts to Step S314.

As described above, according to the second embodiment, the receiving apparatus 23 automatically confirms that the capsule endoscope 10 has passed through the stomach and arrived at the large intestine; accordingly, it becomes possible to reduce the trouble of work of the health care professional.

### (Modification 2-1)

In the receiving apparatus 23, the above automatic confirmation of arrival at the large intestine based on the feature data of an in-vivo image may be executed any time, starting at the examination stage. In this case, an operation of changing the operation mode of the receiving apparatus 23 from the patient mode to the health care professional mode is performed upon confirmation of arrival at the large intestine, which brings a shift to the post-examination processing stage.

Specifically, when it is confirmed by the determination of the determination unit 232 that the capsule endoscope 10 has arrived at the large intestine, the control unit 210 stores in the memory 209 the time at that point as the large intestine arrival time as well as displaying on the display unit 203 a message to notify that the capsule endoscope 10 has arrived at the large intestine.

A screen W52 illustrated in FIG. 23 is a display example of a large intestine arrival notification screen in Modification 2-1. The screen W52 includes a password input field D51 for switching the operation mode of the receiving apparatus 23 from the patient mode to the health care professional mode, and input keys D52 and icons D53 and D54, which are used for the input of a password. A password is inputted by a touch operation on the screen W52, and the control unit 210 verifies the inputted password. When the verification of the password is successful, the operation mode setting unit 212 switches the operation mode of the receiving apparatus 23 to the health care professional mode. The control unit 210 subsequently executes predetermined processes such as displaying on the display unit 203 a screen to instruct a step to be taken for the patient by the health care professional in the post-examination processing.

### (Modification 2-2)

In the determination unit 232, the excretion of the capsule endoscope 10 out of the body may be confirmed automatically. Specifically, the image processing unit 231 calculates the brightness value of an in-vivo image and the R-value of an RGB color specification system as the feature data of the in-vivo image during the post-examination stage. The determination unit 232 determines whether or not the capsule endoscope 10 has been excreted out of the body by comparing the calculated brightness value and R-value with predetermined threshold values.

When it is determined that the capsule endoscope 10 has been excreted out of the body, the control unit 210 stores the time as the excretion time in the memory 209. At this point, the control unit 210 may notify the health care professional or patient that the capsule endoscope 10 has been excreted out of the body, such as by generating sound and vibration, and blinking the received image display unit 205 in a predetermined color. Moreover, the receiving apparatus 23 may subsequently stops receiving image data from the capsule endoscope 10 automatically.

### (Third Embodiment)

Next, a description will be given of a third embodiment of the present invention.

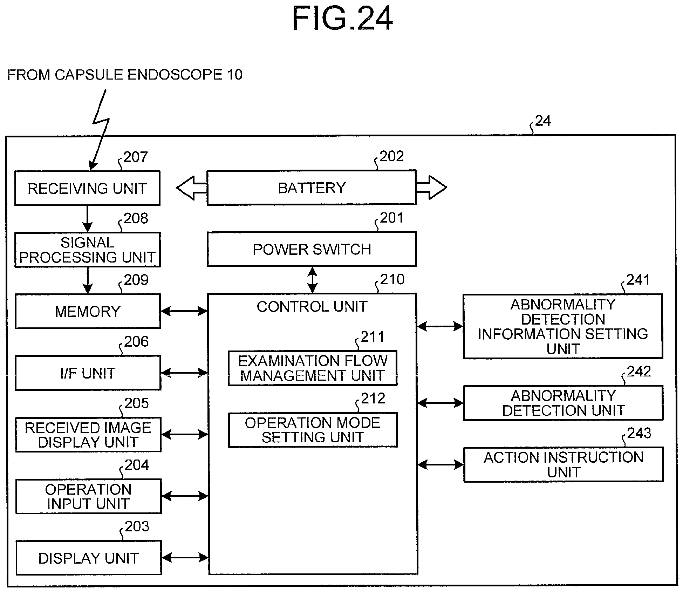

FIG. 24 is a block diagram illustrating the configuration of a receiving apparatus according to the third embodiment. The third embodiment is characterized in that the receiving apparatus notifies an action instruction to the patient when an abnormal situation occurs during the examination. The entire configuration of a capsule endoscope system in the third embodiment is similar to the one illustrated in FIG. 1.

As illustrated in FIG. 24, a receiving apparatus 24 according to the third embodiment includes an abnormality detection information setting unit 241, an abnormality detection unit 242, an action instruction unit 243, in addition to the configuration of the receiving apparatus 20 (FIG. 3). The configurations and operations of the other units are similar to those illustrated in FIG. 3.

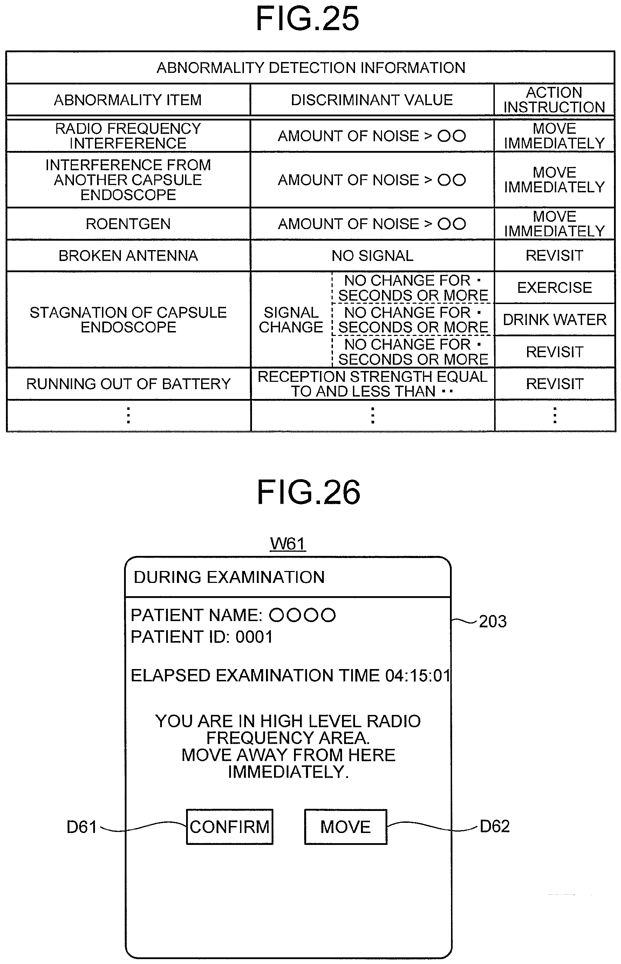

The abnormality detection information setting unit 241 sets abnormality detection information where an item of an abnormal situation that can occur in the capsule endoscope 10 or the receiving apparatus 24 during the examination (abnormality item), a threshold value (discriminant value) for determining an abnormality, and an action to make the patient execute when an abnormality occurs (action instruction) are associated. FIG. 25 is a table illustrating an example of the abnormality detection information stored in the memory 209. As illustrated in FIG. 25, the abnormality detection information contains radio frequency interference, the stagnation of the capsule endoscope 10 that the patient swallowed, and the like, as the abnormality items. A discriminant value and an action instruction are set for each of these abnormality items. The discriminant value is set based on the health care professional's experience, the result of a past statistic analysis, and the like.

Such abnormality detection information may be set by being inputted by the health care professional directly into the receiving apparatus 24, or may be set by transferring information edited in the control apparatus 30 and other devices to the receiving apparatus 24. Moreover, the abnormality detection information may be set by default, or may be set at the time of the initialization of the receiving apparatus 24 or at any other arbitrary timings. Furthermore, the abnormality detection information once set can be edited at any time in the receiving apparatus 24.

The abnormality detection unit 242 detects the occurrence of an abnormality by monitoring the strength of a wireless signal that the receiving unit 207 receives from the capsule endoscope 10, the signal conditions (the amount of noise, and the like), and other various physical quantities, and comparing them with the discriminant values of the abnormality detection information.

For example, when the patient enters a high level radio frequency area during the examination, radio frequency interference occurs between the capsule endoscope 10 and the receiving apparatus 24. In such a case, the abnormality detection unit 242 detects the amount of noise in a wireless signal received from the capsule endoscope 10 as well as detecting the occurrence of an abnormality by comparing the detected value with the discriminant value of the abnormality detection information. Otherwise, when the capsule endoscope 10 stagnates in the body of the patient, there is less change in wireless signals received by the receiving antennas 21a to 21h. In such a case, the abnormality detection unit 242 detects change in the strength of signals of the receiving antennas 21a to 21h and, when there is no change in the strength of signals for a predetermined time or more, detects the occurrence of an abnormality.

The action instruction unit 243 extracts an action instruction corresponding to an abnormality item from the abnormality detection information when the abnormality detection unit 242 detects the occurrence of the abnormality.

When the abnormality detection unit 242 detects the abnormality, the control unit 210 displays on the display unit 203 a screen to give the patient the action instruction extracted by the action instruction unit 243. A screen W61 illustrated in FIG. 26 is a display example of such an action instruction screen, and illustrates a case of instructing the patient to move immediately when radio frequency interference occurs. At this point, the control unit 210 may draw the patient's attention, such as by generating sound and vibration, or blinking an LED light in a predetermined color (e.g., red). Moreover, the control unit 210 stores in the memory 209 the time of occurrence of the abnormality and the contents of the abnormality while associating them with image data acquired at that point.

In accordance with a touch operation on a confirm icon D61 on the screen W61, the control unit 210 stores the patient's action record in the memory 209, setting the acceptance time of the touch as the confirmation time. On the other hand, in accordance with a touch operation on a move icon D62 on the screen W61, the control unit 210 stores the patient's action record in the memory 209, setting the acceptance time of the touch as the movement time.

As described above, according to the third embodiment, when an abnormality occurs during the examination, an action instruction to the patient is displayed on the receiving apparatus 24; accordingly, the patient can have a relaxed free action time. Moreover, it is possible to keep the influence of an abnormal situation on the examination to a minimum by prompting the patient to an appropriate action upon occurrence of an abnormality. Furthermore, the time of occurrence of an abnormality and the contents of the abnormality are stored while being associated with image data. Accordingly, the use of these pieces of information (e.g., deleting an in-vivo image associated with the time of occurrence of an abnormality) makes it possible to promote efficiency in interpretation work.

### (Fourth Embodiment)

Next, a description will be given of a fourth embodiment of the present invention.

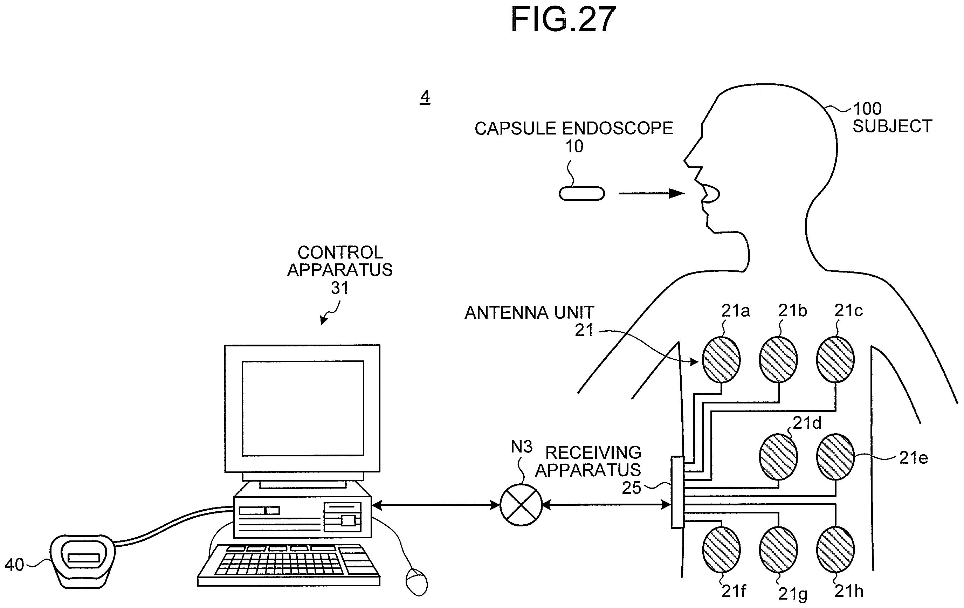

FIG. 27 is a schematic diagram illustrating the schematic configuration of a capsule endoscope system according to the fourth embodiment. This capsule endoscope system 4 includes the capsule endoscope 10, a receiving apparatus 25, and a control apparatus 31, and is characterized in that the receiving apparatus 25 and the control apparatus 31 transmit and receive information to and from each other in wired or wireless communication via a communication network N3. Similarly to the first embodiment, the cradle 40 is used to download image data from the receiving apparatus 25.

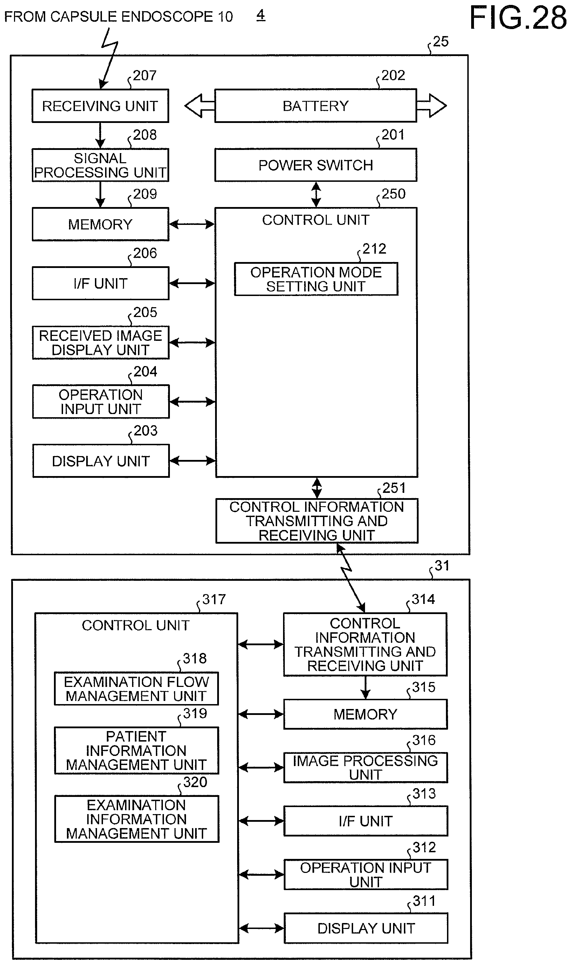

FIG. 28 is a block diagram illustrating the configuration of the capsule endoscope system 4.

In relation to the configuration of the receiving apparatus 20 (FIG. 3), the receiving apparatus 25 includes a control unit 250 having the operation mode setting unit 212, instead of the control unit 210, and further includes a control information transmitting and receiving unit 251.

The control unit 250 controls the operations of the units of the receiving apparatus 25 based on various information received from the control apparatus 31.

The control information transmitting and receiving unit 251 transmits and receives various information directly or indirectly to and from the control apparatus 31 in wired or wireless communication. For example, the control information transmitting and receiving unit 251 transmits information such as elapsed examination time measured by the receiving apparatus 25 to the control apparatus 31 at predetermined intervals. As a specific aspect of such a receiving apparatus 25, a mobile device such as a PDA, mobile phone, or smartphone can be used. The configurations and operations of the other units are similar to those illustrated in FIG. 3.

The control apparatus 31 includes a display unit 311, an operation input unit 312, an interface (I/F) unit 313, a control information transmitting and receiving unit 314, a memory 315, an image processing unit 316, and a control unit 317.

The display unit 311 is realized by a display device such as a CRT display, liquid crystal display, or EL display, and displays various information related to the examination, an in-vivo image, and the like on a screen.

The operation input unit 312 is realized by an input device such as a keyboard, mouse, touch panel, various types of switches, and accepts an input signal in accordance with an operation of the health care professional to input into the control unit 317.

The interface unit 313 includes a connection port with an external device (reading device that reads image data from a portable recording medium, and the like), such as a USB port, and accepts the input of signals representing image data and their related information and the like, which are inputted via the USB port and the like.

The control information transmitting and receiving unit 314 transmits and receives various information directly or indirectly to and from the receiving apparatus 25 in wired or wireless communication.

The memory 315 is realized by semiconductor memory such as flash memory, RAM, or ROM, a recording medium such as an HDD, MO, CD-R, or DVD-R, a drive device that drives the recording medium, and the like. Stored in the memory 315 are programs for causing the control apparatus 31 to operate and execute various functions, image data on which various processes are performed in the image processing unit 316 and their related information, data to be used during execution of the program, and the like. Moreover, the image data received from the receiving apparatus 25 are temporarily stored in the memory 315.

The image processing unit 316 performs image processing such as a white balance process, demosaicing, color conversion, gray scale transformation (such as gamma transformation), smoothing (such as noise reduction), sharpening (such as edge enhancement), on image data transferred from the receiving apparatus 25.

The control unit 317 is realized by hardware such as a CPU, reads various programs stored in the memory 315 to instruct the units configuring the control apparatus 31, transfer data thereto, and the like in accordance with various operation signals inputted via the interface unit 313, and the like, and integrally controls the overall operations of the control apparatus 31. Specifically, the control unit 317 includes an examination flow management unit 318, a patient information management unit 319, and an examination information management unit 320.

The examination flow management unit 318 has examination flow information where processes at stages of the examination are described, and controls the flow of processes in the examination based on the examination flow information. The contents of the examination flow information are similar to those illustrated in FIG. 4.

The patient information management unit 319 manages information related to the patient who takes the examination. The patient information contains information such as patient name, patient ID, date of birth, scheduled examination date, contents of the examination, and the like.

The examination information management unit 320 manages information related to the examination. The examination information contains information such as examination ID, contents of the examination, examination start and end times, various processes performed during the examination, and time required for the respective processes.

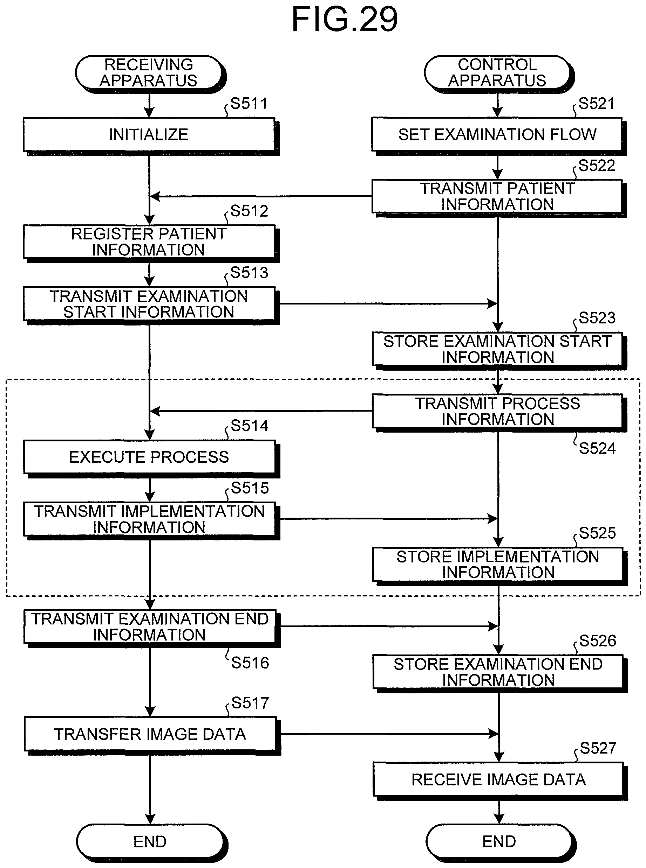

Next, a description will be given of the operations of the capsule endoscope system 4. FIG. 29 is a flowchart illustrating the operations of the receiving apparatus 25 and the control apparatus 31.

Firstly, in Step S511, the receiving apparatus 25 initializes the data accumulated in the memory 209 in accordance with an initialization instruction received from the control apparatus 31, or in accordance with operation input by the user.

On the other hand, in Step S521, the examination flow management unit 318 of the control apparatus 31 sets an examination flow. As the examination flow information, one set by default may be used as it is, or one modified based on information inputted via the operation input unit 312 may be used. In the latter case, the health care professional can add or delete the contents of the processes in accordance with the age, constitutional predisposition, and physique of the patient, the contents of the examination, and the like, or change the time to give an instruction to the patient.

In the subsequent Step S522, the control apparatus 31 transmits patient information related to the patient of the examination target to the receiving apparatus 25. In response to this, the receiving apparatus 25 registers the received patient information (Step S512).

In Step S513, the receiving apparatus 25 transmits examination start information to the control apparatus 31 in accordance with a touch operation on the touch panel 204a. In response to this, the control apparatus 31 stores the examination start information as the examination progress information in the memory 315 (Step S523).

In Step S524, the control apparatus 31 transmits to the receiving apparatus 25 information related to processes to be executed by the health care professional (such as an operation on the receiving apparatus 25 and a step for the patient), the operations of the receiving apparatus 25 associated with these processes, operation modes corresponding to the respective processes, as the process information, in the process order in accordance with the examination flow information. The receiving apparatus 25 executes processes such as the display of a predetermined screen and change in the setting of the operation mode in accordance with the received process information (Step S514).

In Step S515, whenever accepting predetermined operation input specified in the process information (e.g., a touch operation on a predetermined icon performed by the health care professional who has completed one process), the receiving apparatus 25 transmits to the control apparatus 31 implementation information containing the content of the process, and the processed time. In response to this, the control apparatus 31 stores the received implementation information as the examination progress information in the memory 315 (Step S525).

These Steps S514, S515, S524, and S525 are successively executed on a process-by-process basis in accordance with the examination flow information. The details of the processes are similar to those of the first embodiment.

When ending the final process in line with the examination flow, the receiving apparatus 25 transmits examination end information to the control apparatus 31 (Step S516). In response to this, the control apparatus 31 stores the received examination end information as the examination progress information in the memory 315 (Step S526).

When the receiving apparatus 25 is subsequently mounted on the cradle 40, the image data accumulated in the memory 209 of the receiving apparatus 25 are transferred to the control apparatus 31 (Step S517). The control apparatus 31 receives the image data to store in the memory 315 (Step S527).

As described above, in the fourth embodiment, the examination flow is managed by the control apparatus 31, and information related to a predetermined process is transmitted at a predetermined timing to the receiving apparatus 25 to execute the process. Consequently, the health care professional and the patient can take an appropriate action in accordance with the examination flow, such as by referring to a screen displayed on the receiving apparatus 25. Moreover, according to the fourth embodiment, information acquired by the receiving apparatus 25 is transmitted at any time to the control apparatus 31; accordingly, it is also possible to control the progress of the examination on the control apparatus 31 side in accordance with the situation.

Moreover, according to the fourth embodiment, elapsed examination time measured by the receiving apparatus 25 is transmitted regularly to the control apparatus 31; accordingly, even while the patient is out, the health care professional can readily grasp the patient's examination information. Therefore, the health care professional can take measures such as proceeding without delay with reception preparations for the patient's revisit, and the like.

In the fourth embodiment, various information stored as the examination progress information is transmitted from the receiving apparatus 25 to the control apparatus 31. However, these pieces of information may be transmitted to the real time viewer that can communicate with the receiving apparatus 25 by wire or wirelessly to be displayed on the screen. In this case, there will be no need for the health care professional to start the control apparatus 31 during the examination, and it becomes possible to grasp the situation of the examination with a simple configuration in real time.

Moreover, in the fourth embodiment, whenever each process set in the examination flow information is executed, the implementation information is transmitted from the receiving apparatus 25 to the control apparatus 31. However, the implementation information may be accumulated in the memory 209 on the receiving apparatus 25 side. In this case, the implementation information is transferred to the control apparatus 31 together with image data upon download after the end of the examination.

### (Modification 4-1)

Confirmation whether the capsule endoscope 10 swallowed by the patient has passed through the stomach and arrived at the large intestine may be performed on the control apparatus 31 side. As specific operations, firstly, when receiving process information related to the confirmation of passage through the stomach from the control apparatus 31, the receiving apparatus 25 transmits image data acquired at that point from the capsule endoscope 10 to the control apparatus 31. The control apparatus 31 performs image processing on the received image data, calculates the feature data of the associated in-vivo image for comparison with the threshold value, and accordingly determines whether the in-vivo image is one after passage through the stomach. Otherwise, the health care professional may visually determine the in-vivo image displayed on the display unit 311. At this point, when the in-vivo image is one after passage through the stomach, the control apparatus 31 transmits the next process information to the receiving apparatus 25. On the other hand, when the in-vivo image has not arrived at the stomach yet, the control apparatus 31 transmits to the receiving apparatus 25 process information related to an additional process to promote the peristaltic movement of the patient. Also with respect to confirmation of arrival at the large intestine, image data may similarly be transmitted from the receiving apparatus 25 to the control apparatus 31 to be determined on the control apparatus 31 side.

### (Modification 4-2)

In the fourth embodiment, the operation mode setting unit 212 of the receiving apparatus 25 sets the operation mode in accordance with process information transmitted from the control apparatus 31. However, control of the operation mode may be performed directly on the control apparatus 31 side. Specifically, the control apparatus 31 transmits an operation mode setting signal to the receiving apparatus 25 in accordance with an operation signal inputted from the operation input unit 312. The receiving apparatus 25 sets the operation mode of the receiving apparatus 25 to the health care professional mode or patient mode in accordance with the received operation mode setting signal.

According to Modification 4-2, the health care professional can switch the operation mode of the receiving apparatus 25 manually. Therefore, it becomes possible to set the operation mode of the receiving apparatus 25 as circumstances demand in accordance with the situation of the examination. Moreover, the operation mode of the receiving apparatus 25 is controlled by remote operation from the control apparatus 31; accordingly, it is possible to prevent situations such as cancelling the health care professional mode by an operating error, and being impossible to cancel the health care professional mode due to a mistake in the input of a password.

### (Modification 4-3)

The setting of the operation mode in the receiving apparatus 25 may be configured to be switched in accordance with the coming and going of the patient to and out of the hospital. Specifically, while the patient carrying the receiving apparatus 25 is in the hospital, in other words, while the receiving apparatus 25 can be connected to a wireless network constructed in the hospital, the operation mode of the receiving apparatus 25 is set to health care professional mode. On the other hand, while the patient is away from the hospital, in other words, while the receiving apparatus 25 is disconnected from the wireless network in the hospital, the operation mode of the receiving apparatus 25 is set to the patient mode.

### (Modification 4-4)

In the fourth embodiment, the patient performs operation input on the receiving apparatus 25 to store information on a revisit (corresponding to Step S212 in FIG. 8), similarly to the first embodiment. However, it may be configured such that when the patient passes the reception desk of the hospital, his/her revisit is accepted automatically.

For example, an intra-hospital wireless network connected to the control apparatus 31 is constructed in the reception area of the hospital. The patient carrying the receiving apparatus 25 enters the area where the receiving apparatus 25 can connect to the intra-hospital wireless network, the receiving apparatus 25 transmits revisit information to the control apparatus 31. In response to this, the control apparatus 31 stores the revisit information in the memory 315 as well as extracting the patient information, and transmits process information related to an action to be subsequently executed by the patient to the receiving apparatus 25 via the wireless network.

Otherwise, a reception terminal that can communicate with the receiving apparatus 25 wirelessly may be installed at the reception desk of the hospital to transmit revisit information from the receiving apparatus 25 to the control apparatus 31 via the reception terminal.

### (Modification 4-5)

In the fourth embodiment, the examination flow management unit 318 controls the flow of processes from the examination preparation to the post-examination processing. However, similarly to Modification 1-6, the flow of processes in the patient's pre-examination schedule may be controlled using the receiving apparatus 25. In this case, the examination flow management unit 318 transmits examination flow information that information related to processes and actions to be executed before the examination is added to the examination flow information illustrated, for example, in FIG. 4 in advance to the receiving apparatus 25 via the control information transmitting and receiving unit 314. Moreover, the patient's action record before the examination may be accumulated in the memory 209 of the receiving apparatus 25 to be transferred to the control apparatus 31 upon download of image data, or may be transmitted from the receiving apparatus 25 to the control apparatus 31 whenever operation input is performed on the receiving apparatus 25, and stored in the memory 315 of the control apparatus 31.

### (Modification 4-6)

In the fourth embodiment, after receiving the implementation information related to confirmation of passage through the stomach and arrival at the large intestine, and the like from the receiving apparatus 25, the control apparatus 31 may use these pieces of implementation information for the management of the subsequent examination flow.

For example, when it is not confirmed that the capsule endoscope 10 has arrived at the large intestine, and the capsule endoscope 10 falls in a stop state (state where the battery of the capsule endoscope 10 has run out, an image cannot be captured, and a wireless signal cannot be transmitted), a reexamination is necessary. This is because when the capsule endoscope 10 stops before confirmation of arrival at the large intestine, the entire area of the small intestine may not be captured.

Hence, when the control apparatus 31 receives from the receiving apparatus 25 information related to the stop of the capsule endoscope 10 prior to information on confirmation of arrival at the large intestine, it is determined that a reexamination is necessary. The examination information management unit 320 then searches for already registered examination information, extracts a plurality of examination dates when the reexamination is possible, and transmits the examination dates to the receiving apparatus 25. In response to this, the receiving apparatus 25 displays the extracted examination dates on the display unit 203. The patient can select the examination date when he/she can come from among the plurality of examination dates displayed on the display unit 203. The receiving apparatus 25 transmits the result of the patient's selection (e.g., touch on a part where a desired examination date is displayed on the display unit 203) to the control apparatus 31.

### (Modification 4-7)

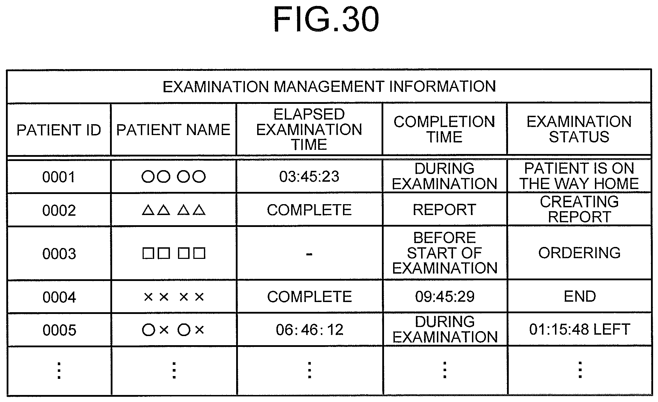

The examination progress information that the control apparatus 31 receives from the receiving apparatus 25 can be used, for example, for the management of examinations for a plurality of patients. FIG. 30 is a table illustrating examination management information created by collecting individual examination progress information. The examination management information contains information such as patient information such as patient IDs and patient names, elapsed examination time in each patient's examination, completion times of the examinations, and current examination statuses.

The health care professional can grasp situations of a plurality of examinations, and take measures in accordance with the situations by referring to such examination management information. Specifically, the health care professional can refer to the remaining time until the end of the examination, which is described in the examination status, (e.g., "01:15:48 left") and start a preparation for the acceptance of the patient who will revisit, and the like.

Moreover, the examination management information collected in this manner is processed statistically to make it possible to be utilized for the creation of examination schedules of a plurality of patients.

### (Fifth Embodiment)

Next, a description will be given of a fifth embodiment of the present invention.

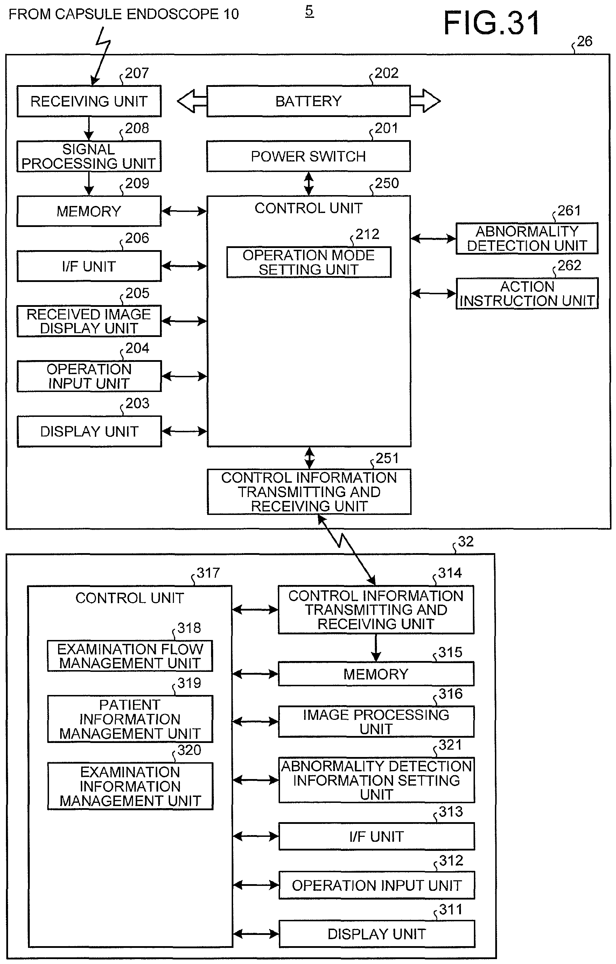

FIG. 31 is a block diagram illustrating the configuration of a capsule endoscope system according to the fifth embodiment. A capsule endoscope system 5 according to the fifth embodiment includes a receiving apparatus 26 and a control apparatus 32, and is characterized in that the control apparatus gives an action instruction to the patient via the receiving apparatus when an abnormal situation occurs during the examination.

As illustrated in FIG. 31, the control apparatus 32 includes an abnormality detection information setting unit 321 in addition to the configuration of the control apparatus 31 (FIG. 28). The abnormality detection information setting unit 321 sets abnormality detection information where an item of an abnormal situation that can occur during the examination (abnormality item), a threshold value (discriminant value) for determining an abnormality, and an action to make the patient execute when an abnormality occurs (action instruction) are associated, and transmits the abnormality detection information to the receiving apparatus 26 via the control information transmitting and receiving unit 314. The contents of the abnormality detection information are similar to those illustrated in FIG. 25. The abnormality detection information setting unit 321 may transmit the abnormality detection information set by default, as it is, to the receiving apparatus 26, or may extract abnormality detection information on an examination-by-examination basis from a plurality of types of abnormal detection information generated according to the categories (such as gender and age) of the patient and the contents of an examination, and transmit the abnormal detection information to the receiving apparatus 26. The abnormality detection information setting unit 321 may edit (add, delete, and change) abnormality detection information as appropriate based on information inputted from the operation input unit 312, or may newly generate abnormality detection information based on information inputted from the operation input unit 312.

On the other hand, the receiving apparatus 26 includes an abnormality detection unit 261 and an action instruction unit 262, in addition to the configuration of the receiving apparatus 25 (FIG. 28). The abnormality detection unit 261 detects the occurrence of an abnormality by monitoring the strength of a wireless signal that the receiving apparatus 26 receives from the capsule endoscope 10, the signal conditions (the amount of noise, and the like), and other various physical quantities, and referring to abnormality reference information transmitted from the control apparatus 32 and stored in the memory 209.

The action instruction unit 262 extracts an action instruction corresponding to an abnormality item from the abnormality detection information when the abnormality detection unit 261 detects the occurrence of an abnormality.

For example, when the patient enters a high level radio frequency area during the examination, the abnormality detection unit 261 compares the amount of noise in a wireless signal received from the capsule endoscope 10 with the discriminant value in the abnormality detection information, and detects the occurrence of radio frequency interference. In response to this, the action instruction unit 262 extracts an action instruction corresponding to an action instruction "move immediately." The control unit 250 generates a screen to notify the patient of the extracted action instruction (refer to FIG. 26) to display on the display unit 203. At this point, the control unit 250 may draw the patient's attention, such as by generating sound and vibration, or blinking an LED light in a predetermined color (e.g., red). Otherwise, the control unit 250 may notify the health care professional of the occurrence of an abnormality by automatic transmission to a preset mobile terminal (such as a mobile phone of a health care professional in charge).

Furthermore, the control unit 250 stores in the memory 209 information such as the time of occurrence of an abnormality, the contents of the abnormality, and the record of the subsequent action of the patient. Otherwise, the control unit 250 may transmit these pieces of information to the control apparatus 32 whenever an abnormality occurs.

As described above, according to the fifth embodiment, when an abnormality occurs during the examination, an action instruction to the patient is displayed on the receiving apparatus 26; accordingly, the patient can have a relaxed free action time. Moreover, it is possible to keep the influence of an abnormal situation on the examination to a minimum by prompting the patient to an appropriate action.

Furthermore, according to the fifth embodiment, abnormality detection information is set in the control apparatus 32 and transmitted to the receiving apparatus 26; accordingly, it is possible to modify the abnormality detection information at any time even in the middle of the examination. Therefore, it becomes possible to notify the patient of an action instruction in accordance with the patient's state and action area, and the like as circumstances demand.

### (Modification 5-1)

A screen to be displayed on the display unit 203 when the receiving apparatus 26 detects an abnormality (e.g., the screen W61 of FIG. 26) may be generated on the control apparatus 32 side. Specifically, when the abnormality detection unit 261 detects an abnormality, the receiving apparatus 26 transmits to the control apparatus 32 information such as the occurrence of the abnormality, the value of a physical property to constitute its grounds (e.g., the amount of noise in a wireless signal received from the capsule endoscope 10). In response to this, the control apparatus 32 refers to abnormality detection information set by the abnormality detection information setting unit 321 based on the received information, and extracts an abnormality item and an action instruction. Furthermore, the control apparatus 32 generates a screen on which the action instruction to the patient is displayed to transmit to the receiving apparatus 26. The receiving apparatus 26 displays the received screen on the display unit 203.

According to Modification 5-1, it is possible to reduce the load of an operation on the receiving apparatus 26 side.

As means for transmitting a message and a screen from the control apparatus 32 to the receiving apparatus 26, HTTP (Hyper Text Transfer Protocol), FTP (File Transfer Protocol), or the like may be used. Moreover, it is possible to use a general application and a dedicated application, in addition to email software (mailer), upon display of a message and a screen, which are received in the receiving apparatus 26.

### (Modification 5-2)

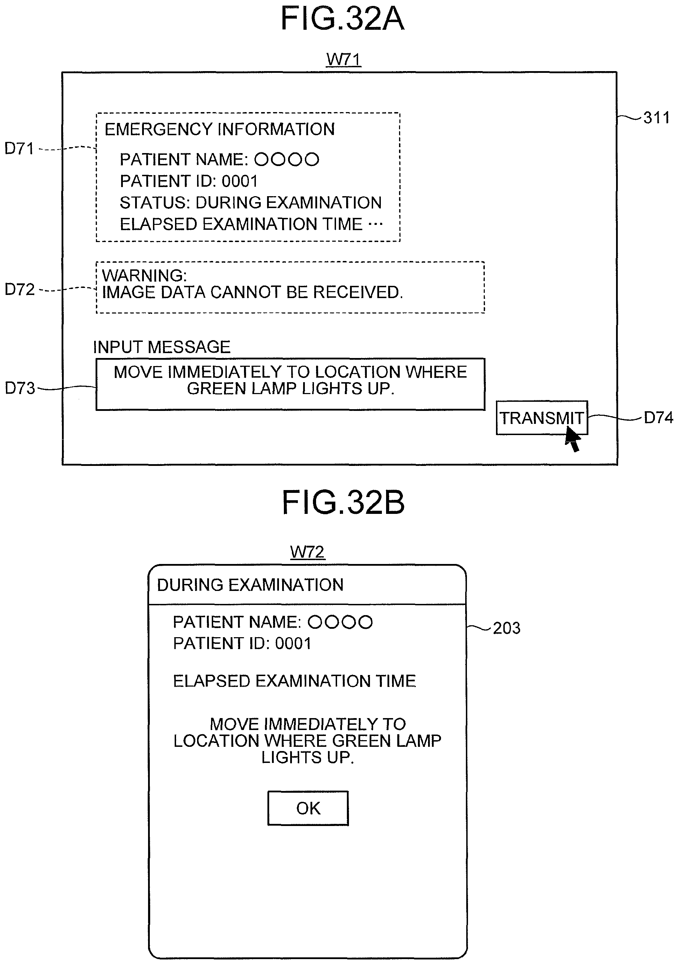

A message to be displayed on the display unit 203 when the receiving apparatus 26 detects an abnormality may be created manually on the control apparatus 32 side. Specifically, when the abnormality detection unit 261 detects an abnormality, the receiving apparatus 26 transmits to the control apparatus 32 information such as the occurrence of the abnormality, and the value of a physical property to constitute its grounds. In response to this, the control apparatus 32 displays on the display unit 311 a screen to notify the health care professional of the occurrence of the abnormality. A screen W71 illustrated in FIG. 32A is a display example of such a notification screen. The screen W71 includes an emergency information display area D71 to display information such as patient information corresponding to an examination where the abnormality occurs, and the examination status, a warning display area D72 to display the contents of the abnormality, and a text input area D73 where the health care professional inputs a message to the patient. When an operation to input text in the text input area D73 and a pointer operation to select a transmission icon D74 using a mouse and the like are performed on the screen W71, the control apparatus 32 transmits a message written in the text input area D73 to the receiving apparatus 26.

In response to this, the receiving apparatus 26 displays on the display unit 203 a screen on which the message received from the control apparatus 32 is displayed. A screen W72 illustrated in FIG. 32B is a display example of such a message screen. The patient can look at such a screen W72, and act following the message (action instruction) displayed thereon.

According to Modification 5-2, even if the unexpected happens to the patient during free actions, it is possible to notify the patient of an appropriate action instruction in accordance with the situation.

### (Modification 5-3)

In the fifth embodiment, when an abnormal situation occurs, an action instruction to the patient is simply displayed on the display unit 203 of the receiving apparatus 26. However, it may be set to enable the patient side to input information into the receiving apparatus 26.

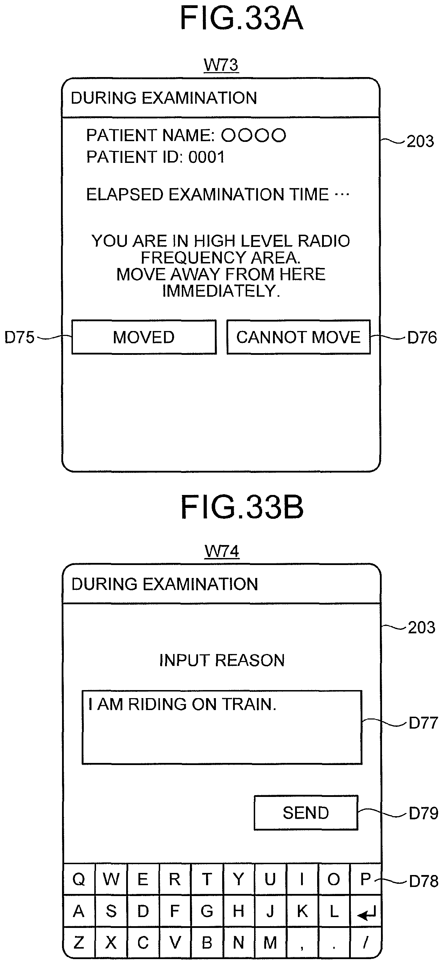

FIGS. 33A and 33B illustrate that when the patient cannot execute a given action instruction, the reason can be inputted. A screen W73 illustrated in FIG. 33A is a display example of a screen to display an action instruction to the patient. The screen W73 includes an execution icon (icon that is displayed as "moved" in FIG. 33A) D75 to be touched when the patient executes the instructed action, a non-execution icon (icon that is displayed as "cannot move" in FIG. 33A) D76 to be touched when the patient cannot execute the instructed action.

When the execution icon D75 is selected by a touch operation on the screen W73, the receiving apparatus 26 stores in the memory 209 the acceptance time of the touch as the execution time of the displayed instruction. On the other hand, when the non-execution icon D76 is selected on the screen W73, the receiving apparatus 26 displays on the display unit 203 a screen to have the patient input the reason why she/he cannot execute the instructed action. A screen W74 illustrated in FIG. 33B is a display example of such a reason input screen. The screen W74 includes a text input area D77 for inputting a message such as a reason, character input keys D78 used when text is inputted, and a send icon D79. The patient can input the reason why he/she cannot execute the instructed action in the text input area D77, using the character input keys D78. Moreover, the patient may input an action taken by him/her together, instead of the instructed action.

In accordance with a touch operation on such a screen W74, the receiving apparatus 26 transmits the message inputted in the text input area D77 to the control apparatus 32. In response to this, the control apparatus 32 stores the received message as the patient's action record in the memory 315. Consequently, the health care professional can grasp the action of the patient upon occurrence of an abnormality in more detail. When a message cannot be transmitted for some reason (e.g., radio frequency interference), the receiving apparatus 26 may store the message in the memory 209, and retransmit the message when in a transmittable state again.

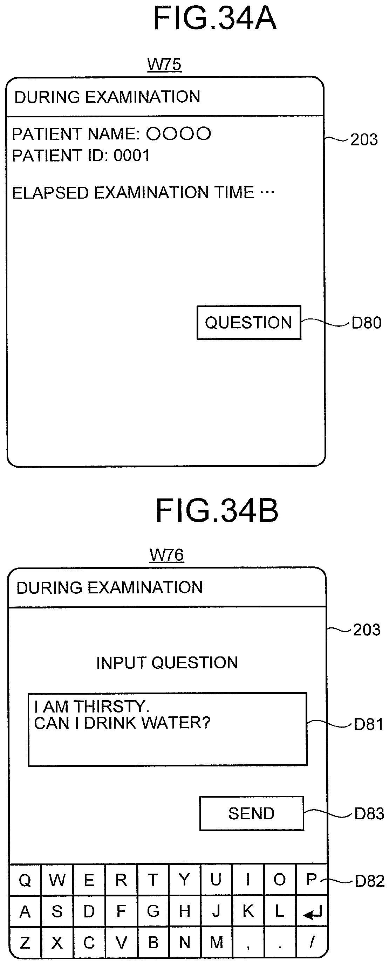

FIGS. 34A to 34D illustrate display examples of the display unit 203 of when the receiving apparatus 26 has the configuration that can transmit a question of the patient during free actions. A screen W75 illustrated in FIG. 34A is a display example of a screen to be displayed on the display unit 203 during the examination (patient mode). The screen W75 includes a question icon D80 that the patient touches at any time when the patient has a question.

In accordance with a touch operation on the question icon D80 on the screen W75, the receiving apparatus 26 displays on the display unit 203 a screen to allow the patient to input a question. FIG. 34B is a display example of such a question input screen. The screen W76 includes a text input area D81 for inputting a question, character input keys D82 used upon input of text, and a send icon D83.

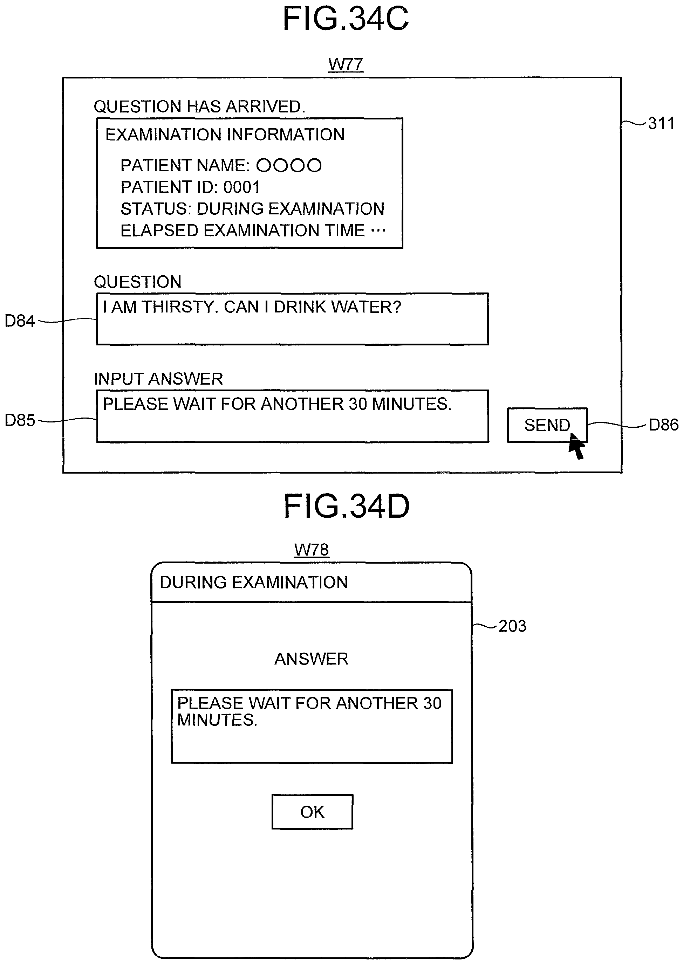

In accordance with a touch operation on such a screen W76, the receiving apparatus 26 transmits a question inputted in the text input area D81 to the control apparatus 32. In response to this, the control apparatus 32 displays on the display unit 311 a screen to display the question inputted in the text input area D81. A screen W77 illustrated in FIG. 34C is a display example of such a question display screen. The screen W77 includes a question display area D84 to display a question from the patient, a text input area D85 for inputting the answer, and a send icon D86. The health care professional can create an answer and an appropriate action instruction to the patient, using an input device such as a keyboard, for the question from the patient displayed on the screen W77.

When an operation to input text in the text input area D85 and a pointer operation to select the send icon D86 are performed on the screen W77, the control apparatus 32 transmits an answer inputted in the text input area D85 to the receiving apparatus 26. In response to this, the receiving apparatus 26 displays a screen to display the received answer on the display unit 203. A screen W78 illustrated in FIG. 34D is a display example of the answer display screen. The patient can act following the answer displayed on such a screen W78.

In this manner, according to Modification 5-3, even if the unexpected happens to the patient during free actions, it is possible to grasp the patient's specific action and covey an appropriate action instruction to the patient.

In Modification 5-3, text documents are transmitted and received between the receiving apparatus 26 and the control apparatus 32 to convey messages between the patient and the health care professional. However, messages can be conveyed between both by other means, or by combining the means with the other means. For example, the receiving apparatus 26 and the control apparatus 32 may be configured such that voice data can be transmitted and received to and from each other to accept and answer questions by voice between the patient and the health care professional.

### (Modification 5-4)

In the fifth embodiment, the flow of the processes from the examination preparation to the post-examination processing is controlled. However, similarly to Modification 4-5, the flow of processes in the patient's pre-examination schedule may be controlled using the receiving apparatus 26. In this case, even at the pre-examination stage, a message to the patient may be created manually to be transmitted at any time from the control apparatus 32 to the receiving apparatus 26 similarly to Modification 5-2, or a message and a question from the patient may be transmitted at any time from the receiving apparatus 26 to the control apparatus 32 similarly to Modification 5-3. Moreover, medical interview information such as the physical condition of the patient before the examination may be transmitted and received between the receiving apparatus 26 and the control apparatus 32. Furthermore, the control apparatus 32 may be set to notify the coming of the execution times of processes and actions to be executed by the patient to a contact address (such as an email address of a mobile terminal) preregistered in the patient information.

### (Sixth Embodiment)

Next, a description will be given of a sixth embodiment of the present invention.

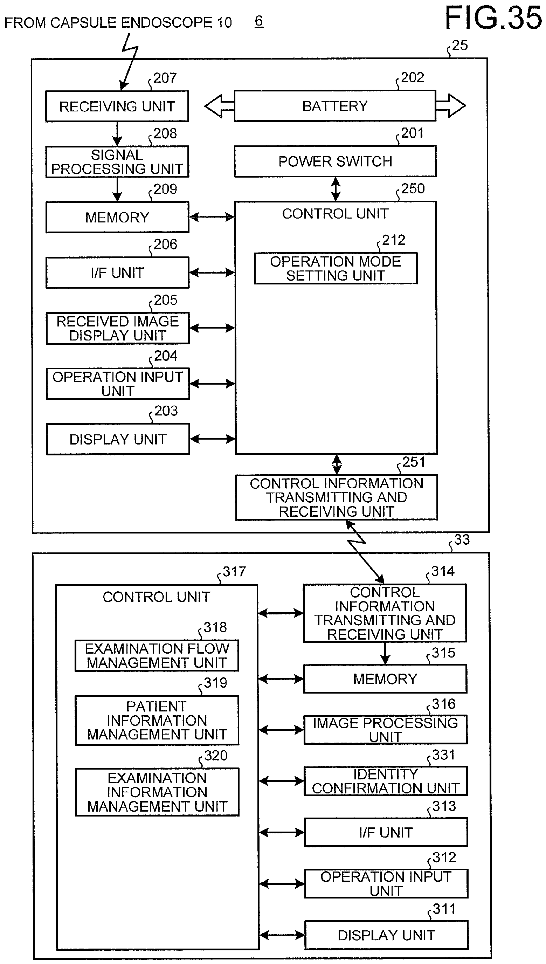

FIG. 35 is a block diagram illustrating a capsule endoscope system according to the sixth embodiment. As illustrated in FIG. 35, a capsule endoscope system 6 according to the sixth embodiment includes the receiving apparatus 25 and a control apparatus 33, and is characterized in that the capsule endoscope system 6 automatically confirms the identity of the patient who takes the examination.

As illustrated in FIG. 35, the control apparatus 33 includes an identity confirmation unit 331 in addition to the configuration of the control apparatus 31 (FIG. 28). When receiving image data that an image of the face of the patient is captured from the receiving apparatus 25, the identity confirmation unit 331 refers to the patient's face image data preregistered by the patient information management unit 319 and stored in the memory 315, and confirms the identity of the patient. The other configurations and operations of the control apparatus 33 are similar to those of the control apparatus 31 illustrated in FIG. 28.

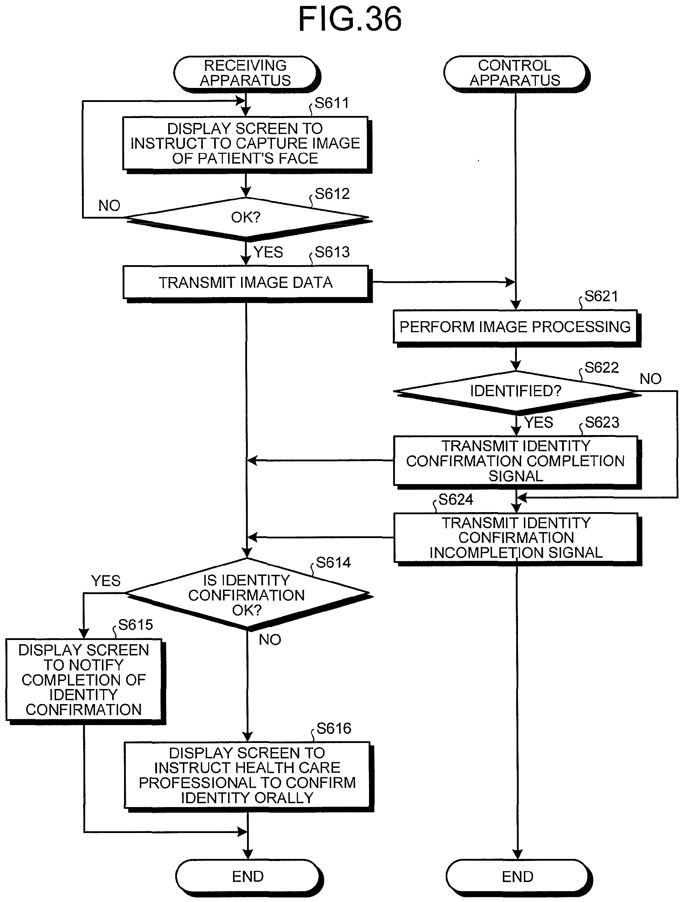

Next, a description will be given of the operations of the capsule endoscope system according to the sixth embodiment. In the sixth embodiment, with respect to the examination flow information (refer to FIG. 4), the examination flow management unit 318 confirms that the power of the capsule endoscope 10 is on, and that an image has been received in the receiving apparatus 25, and subsequently sets a process of confirming the patient's identity. FIG. 36 is a flowchart illustrating the operations of the receiving apparatus 25 and the control apparatus 33 in the patient's identity confirmation process.

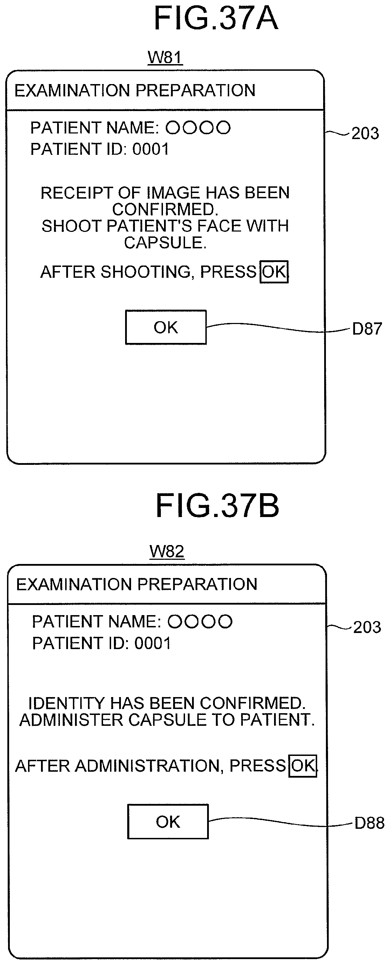

Firstly, in Step S611, the receiving apparatus 25 displays on the display unit 203 a screen to instruct the health care professional to capture an image of the face of the patient. A screen W81 illustrated in FIG. 37A is a display example of such an instruction screen.

When the health care professional aims the capsule endoscope 10 at the patient's face in accordance with the display of the screen W81, the capsule endoscope 10 captures an image of the patient's face that comes within the field of view at predetermined time intervals and transmits the image data wirelessly to the receiving apparatus 25. When an OK icon D87 on the screen W81 is touched (Step S612: Yes), the receiving apparatus 25 transmits to the control apparatus 33 the image data received from the capsule endoscope 10 (Step S613). On the other hand, when the OK icon D87 is not touched (Step S612: No), the receiving apparatus 25 repeats the display of the screen W81.

In Step S621, the image processing unit 316 of the control apparatus 33 performs predetermined image processing on the received image data to generate a face image. In the subsequent Step S622, the identity confirmation unit 331 compares the image data (received image) of the patient's face, on which image processing is performed, with the image (registered image) of the patient's face stored in the memory 315 to confirm the identity. Specifically, the identity confirmation unit 331 determines whether or not the received image matches the registered image by a pattern matching process or the like.

When the received image matches the registered image (Step S622: Yes), the identity confirmation unit 331 transmits an identity confirmation completion signal to the receiving apparatus 25 (Step S623). On the other hand, when the received image does not match the registered image (Step S622: No), the identity confirmation unit 331 transmits an identity confirmation incompletion signal to the receiving apparatus 25 (Step S624).

When receiving the identity confirmation completion signal, the receiving apparatus 25 determines that the control apparatus 33 has confirmed the identity (Step S614: Yes), and displays on the display unit 203 a screen to notify the health care professional that the identity confirmation is complete (Step S615). A screen W82 illustrated in FIG. 37B is a display example of such a notification screen. When an OK icon D88 is touched on the screen W82, the receiving apparatus 25 executes the next process in accordance with the examination flow (refer to FIG. 4).

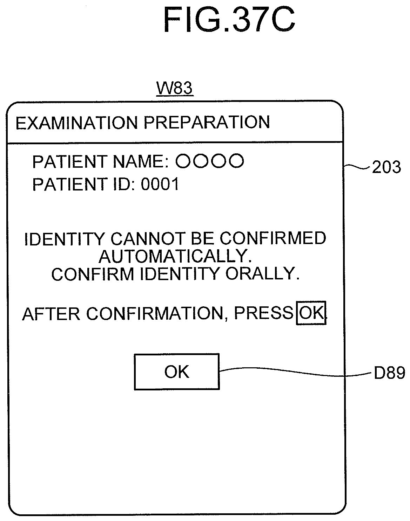

On the other hand, when receiving the identity confirmation incompletion signal, the receiving apparatus 25 determines that the control apparatus 33 has not confirmed the identity (Step S614: No), notifies the health care professional that the identity cannot be confirmed, and displays on the display unit 203 a screen to instruct the health care profession to confirm the identity orally (Step S616). A screen W83 illustrated in FIG. 37C is a display example of such a notification screen. When an OK icon D89 is touched on the screen W83, the receiving apparatus 25 executes the next process in accordance with the examination flow (refer to FIG. 4).

As described above, according to the sixth embodiment, the identity is confirmed automatically based on the patient's face image captured by the capsule endoscope 10; accordingly, it is possible to save the health care professional's trouble and reduce the chances of forgetting to confirm the identity and misidentification.

### (Modification 6-1)

Such an automatic identity confirmation may be performed upon checking consistency between the image data of in-vivo images and patient information when the health care interprets the in-vivo images. Specifically, after the end of the examination, the receiving apparatus 25 transfers to the control apparatus 33 the image data of the patient's face captured by the capsule endoscope 10, together with the image data of the other in-vivo images. The image processing unit 316 performs face authentication on image data in the vicinity of the beginning to extract an image where the face is shot. Moreover, the image processing unit 316 performs predetermined image processing on a series of image data other than that to generate in-vivo images. The identity confirmation unit 331 compares the image extracted by the image processing unit 316 with the patient's face image registered in the patient information management unit 319 to confirm the identity.

It is desired from the viewpoint of personal information protection to delete the patient's face image in the control apparatus 33 after executing the automatic identity confirmation process. Otherwise, a flag indicating to be personal information may be added to the patient's face image to set the patient's face image to non-display, replace it with another image, or indicate explicitly to be an image that is not targeted for interpretation.

### (Seventh Embodiment)

Next, a description will be given of a seventh embodiment of the present invention.

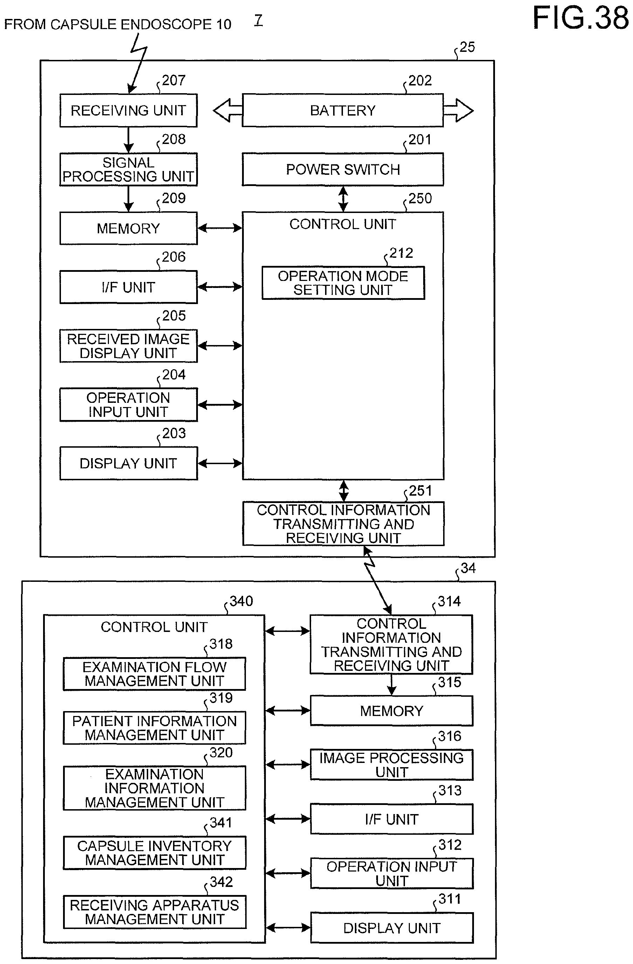

FIG. 38 is a block diagram illustrating the configuration of a capsule endoscope system according to the seventh embodiment. As illustrated in FIG. 38, a capsule endoscope system 7 according to the seventh embodiment includes the receiving apparatus 25 and a control apparatus 34, and is characterized in that the control apparatus 34 manages the inventories of the capsule endoscope 10 and the receiving apparatus in a facility such as a hospital.

As illustrated in FIG. 38, the control apparatus 34 includes a control unit 340 that further has a capsule inventory management unit 341 and a receiving apparatus management unit 342, instead of the control unit 317 in the control apparatus 31 (FIG. 28). The other configurations and operations of the control apparatus 34 are similar to those of the control apparatus 31 illustrated in FIG. 28.

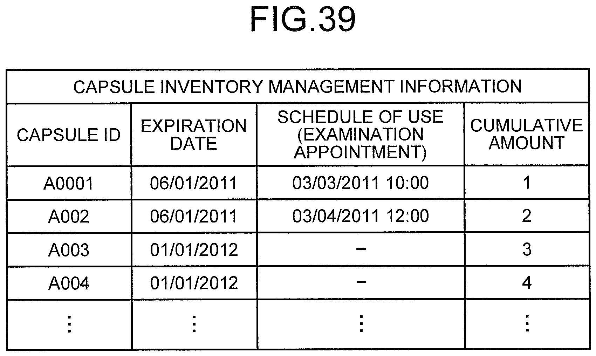

The capsule inventory management unit 341 manages the inventory of the capsule endoscope 10 that the facility owns, based on capsule inventory management information illustrated in FIG. 39, for example. The capsule inventory management information contains information such as IDs, expiration dates, scheduled dates of use (dates of examination appointments), and the quantity of the inventory (cumulative quantity) of the capsule endoscope 10. The health care professional uses the operation input unit 312 to input basic information (such as ID and expiration date), and accordingly, the capsule inventory management information is generated and stored in the memory 315. The capsule inventory management unit 341 manages such capsule inventory management information in conjunction with the examination information management unit 320. Specifically, when examination information managed by the examination information management unit 320 is updated, the capsule inventory management unit 341 receives the updated examination information from the examination information management unit 320 to update the capsule inventory management information based on the examination information.

For example, when a new examination appointment is registered in the examination information, the capsule inventory management unit 341 registers the scheduled date of use of the capsule endoscope 10 in the capsule inventory management information.

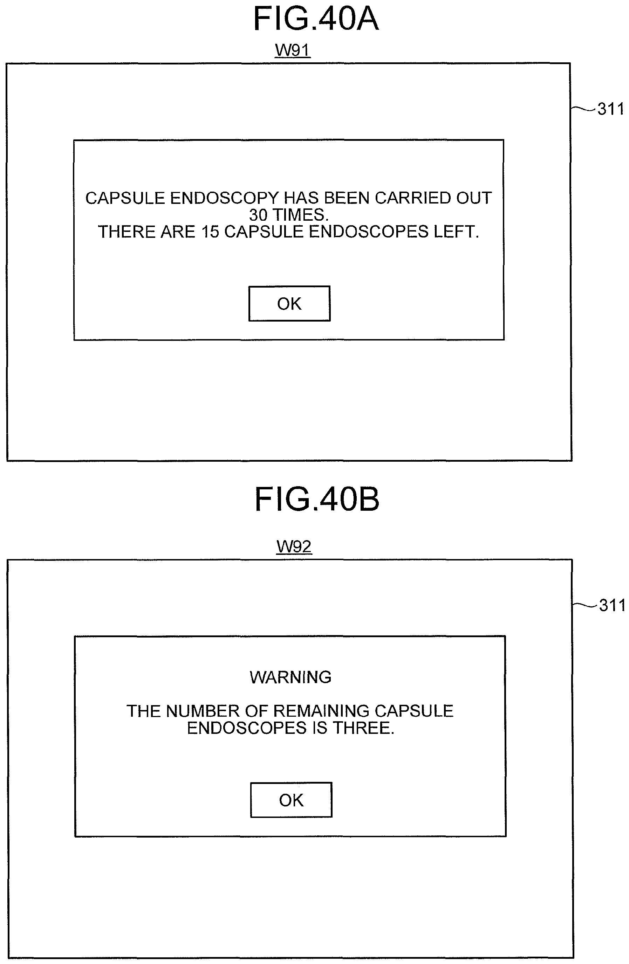

Moreover, when the examination starts, the capsule inventory management unit 341 updates the capsule inventory management information as well as counting the quantity of the inventory of the capsule endoscope 10, and displays on the display unit 311 a screen W91 to notify a current quantity of the inventory as illustrated in FIG. 40A, for example. The quantity of the inventory may be calculated by counting the number of examinations carried out and subtracting it from the number of orders of the capsule endoscope 10.

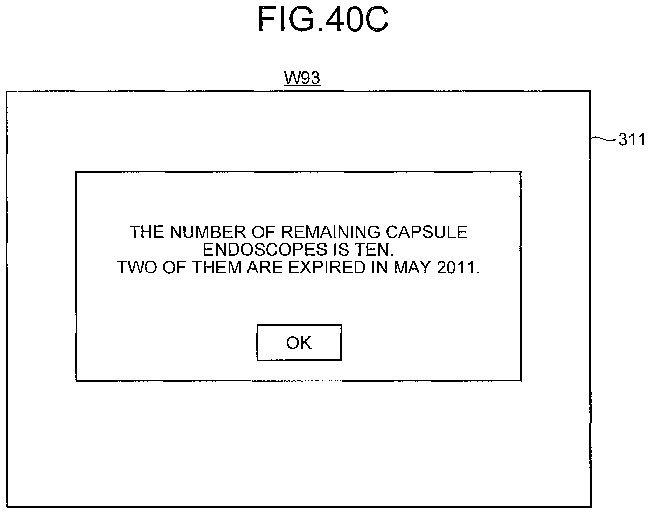

At this point, when the quantity of the inventory of the capsule endoscope 10 is less than a predetermined number, the capsule inventory management unit 341 displays on the display unit 311 a screen W92 to warn of a reduction in the quantity of the inventory as illustrated in FIG. 40B, for example. Moreover, when there is the capsule endoscope 10 whose expiration date is approaching, the capsule inventory management unit 341 displays on the display unit 311 a screen W93 to notify the expiration date as illustrated in FIG. 40C, for example.

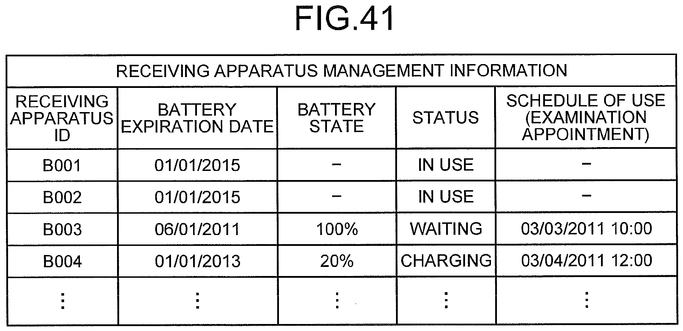

On the other hand, the receiving apparatus management unit 342 manages the receiving apparatuses 25 that the facility owns based on receiving apparatus management information illustrated in FIG. 41, for example. The receiving apparatus management information contains information such as IDs of the receiving apparatuses 25, the expiration dates of batteries, the states of the batteries, statuses, and scheduled dates of use (dates of examination appointments). The health care professional uses the operation input unit 312 to input basic information (such as ID and expiration date of the battery), and accordingly, the receiving apparatus management information is generated and stored in the memory 315. The receiving apparatus management unit 342 manages such receiving apparatus management information in conjunction with the examination information management unit 320. Specifically, when examination information managed by the examination information management unit 320 is updated, the receiving apparatus management unit 342 receives the updated examination information from the examination information management unit 320 to update the receiving apparatus management information based on the examination information.

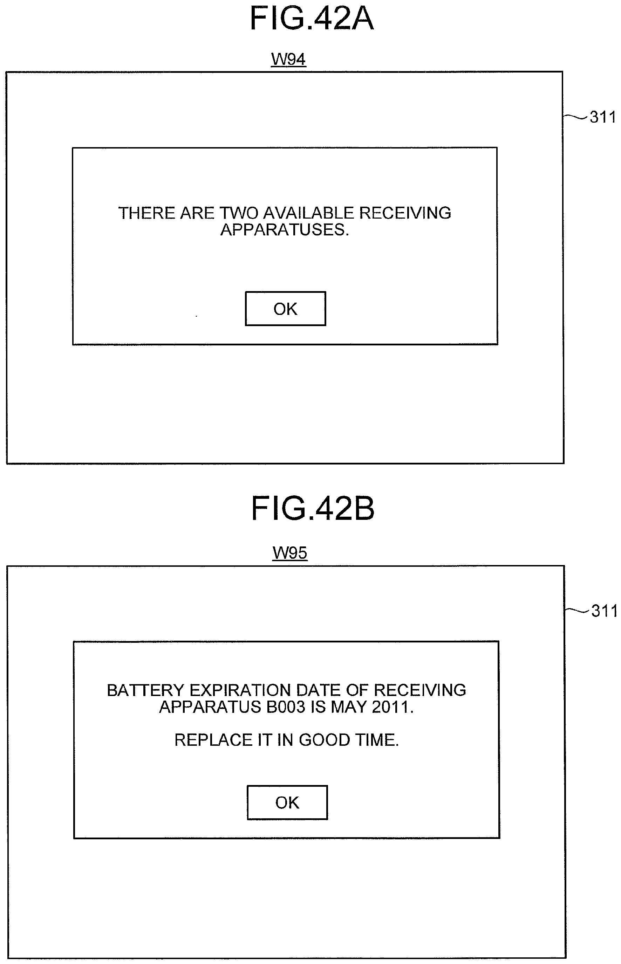

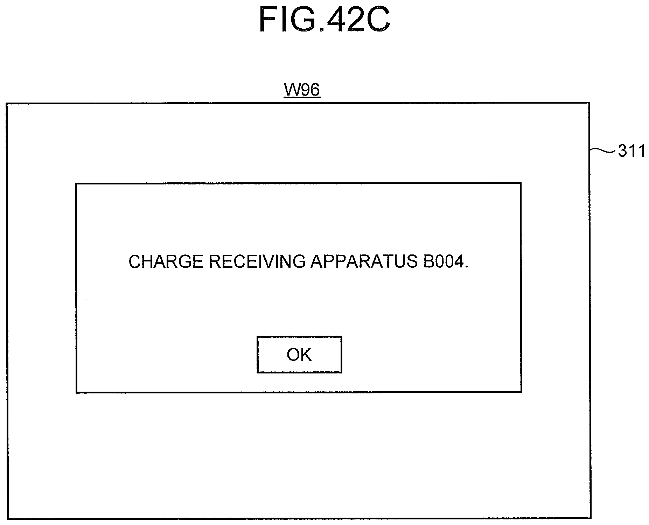

For example, when a new examination appointment is registered in the examination information, the receiving apparatus management unit 342 registers the scheduled date of use of the receiving apparatus 25 in the receiving apparatus management information. Moreover, the receiving apparatus management unit 342 searches the memory 315 for the use of the receiving apparatuses 25, and displays on the display unit 311 a screen W94 to notify the availability of the receiving apparatuses 25 as illustrated in FIG. 42A, for example. At this point, when there is the receiving apparatus 25 whose expiration date of the battery is approaching, the receiving apparatus management unit 342 displays on the display unit 311 a screen W95 to instruct the replacement of the battery as illustrated in FIG. 42B, for example. Furthermore, when the scheduled date of use of any of the receiving apparatuses 25 is approaching, the receiving apparatus management unit 342 displays on the display unit 311 a screen W96 to instruct the charge of the battery as illustrated in FIG. 42C, for example.

As described above, according to the seventh embodiment, the inventory of the capsule endoscope 10 and the use of the receiving apparatus 25 are managed in conjunction with the examination information to ensure preparations for the examination.

Moreover, according to the seventh embodiment, the expiration date of the capsule endoscope 10 can be managed; accordingly, it is possible to avoid waste such as that the expiration date comes, and the unused capsule endoscope 10 is discarded.

### (Modification 7-1)

When an inventory management system and an ordering system of disposable products such as the capsule endoscope 10 is provided in the facility, the capsule inventory management unit 341 and the receiving apparatus management unit 342 may be caused to cooperate with this inventory management system to grasp the quantity of the inventory of the capsule endoscope 10 and the number of the receiving apparatuses held, on the inventory management system side. In this case, it is possible to check the quantity of the inventory of the capsule endoscope 10, and the like without starting the control apparatus 34. Moreover, when the quantity of the inventory of the capsule endoscope 10 falls below a predetermined number, an order may be placed automatically from the ordering system. Furthermore, when the basic information of the capsule inventory management information and the receiving apparatus management information is read directly from the above inventory management system at the time of the order or delivery of the capsule endoscope 10 and the receiving apparatus 25, then it is possible to save the health care professional's trouble to input the basic information directly.

In the above, the description has been given of the management of the examination flow in a capsule endoscopy. However, it is possible to manage flows of various examinations other than that by the patient using a portable terminal device.

Moreover, the first to seventh embodiments and their respective modifications, which are described above, are merely examples for carrying out the present invention, and the present invention is not limited to these embodiments.

### Reference Signs List

1, 4, 5, 6, 7 capsule endoscope system
10 capsule endoscope
20, 23, 24, 25, 26 receiving apparatus
21 antenna unit
21a-21h receiving antenna
22 cable
30, 31, 32, 33, 34 control apparatus
40, 41 cradle
50 real time viewer
60 download device
61 casing
62 holder
63 communication unit
64 battery unit
65 power code
70 computer
100 subject
201 power switch
202 battery
203, 301, 311, 501 display unit
204, 312, 503 operation input unit
204a touch panel
205 received image display unit
206, 313 interface (I/F) unit
207 receiving unit
208 signal processing unit
209, 315 memory
210, 250, 317, 340 control unit
211, 318 examination flow management unit
212 operation mode setting unit
231, 316 image processing unit
232 determination unit
241, 321 abnormality detection information setting unit
242, 261 abnormality detection unit
243, 262 action instruction unit
251, 314 control information transmitting and receiving unit
319 patient information management unit
320 examination information management unit
331 identity confirmation unit
341 capsule inventory management unit
342 receiving apparatus management unit
502 connecting terminal

## Claims

1. A receiving apparatus (20) for receiving information transmitted wirelessly from a capsule endoscope (10) for insertion into a subject and capturing an in-vivo image of the subject, the receiving apparatus (20) comprising:
an operation input unit (204) that is adapted to accept input of information in the receiving apparatus (20);
a display unit (203) that displays information related to an examination using the capsule endoscope (10),
a memory (209) that is adapted to record examination flow information containing contents of a series of processes that are executed or confirmed by a health care professional or a patient in the examination and operation modes of operating the receiving apparatus (20) by the health care professional or the patient to execute or confirm the respective processes in the series of processes; and
a control unit (210, 250) that is adapted to control control operations of the receiving apparatus (20), wherein the control unit (210, 250) includes:
an examination flow management unit (211) that is adapted to control a flow of a series of processes in the examination based on the examination flow information recorded in the memory (209), **characterised by** further comprising an operation mode setting unit (212) that is adapted to extract from the examination flow information an operation mode associated with a process in a current examination flow controlled by the examination flow management unit (211), and to switch an operation mode of the receiving apparatus (20) between a health care professional operation mode in which the health care professional executes or confirms the processes by operating the receiving apparatus (20) and a patient operation mode in which the patient executes or confirms the processes by operating the receiving apparatus (20) in accordance with the extracted operation mode, and to control the operations of the receiving apparatus (20) in accordance with progress of the examination flow for each operation mode set by the operation mode setting unit (212).

2. The receiving apparatus according to claim 1, wherein the control unit (210, 250) is adapted to display on the display unit (203) information related to a process to be executed or confirmed by the health care professional when the receiving apparatus (20) is set in the health care professional operation mode.

3. The receiving apparatus according to claim 2, wherein when the operation input unit (204) is adapted to perform operation input in accordance with a process in a current examination flow controlled by the examination flow management unit (211) when the receiving apparatus (20) is set in the health care professional operation mode, the examination flow management unit permits a shift to a process corresponding to the operation input.

4. The receiving apparatus according to claim 1, wherein the operation mode setting unit (212) is adapted to switch the operation mode of the receiving apparatus (20) to the patient operation mode in accordance with a process in a current examination flow controlled by the examination flow management unit (211) when the receiving apparatus (20) is set in the health care professional operation mode.

5. The receiving apparatus according to claim 1, wherein when the operation input unit (204) is adapted to perform operation input other than previously permitted operation input when the receiving apparatus (20) is set in the patient operation mode, and the control unit (201) is adapted to disable the operation input.

6. The receiving apparatus according to claim 1, wherein the control unit (210, 250) is adapted to display instruction information to the patient on the display unit (203) in accordance with a process in a current examination flow controlled by the examination flow management unit (211) when the receiving apparatus (20) is set in the patient operation mode.

7. The receiving apparatus according to claim 1, wherein the operation mode setting unit (212) is adapted to switch the operation mode of the receiving apparatus (20) to the health care professional operation mode when the operation input unit (204) performs a predetermined operation input when the receiving apparatus (20) is set in the patient operation mode.

8. A capsule endoscope system (1) comprising:
a capsule endoscope (10) for being inserted into a subject and capturing an in-vivo image of the subject;
the receiving apparatus (20) according to claim 1; and
a control apparatus (30) for transmitting and receiving information in wired or wireless communication to and from the receiving apparatus (20).

9. The capsule endoscope system according to claim 8, further comprising:
an image processing unit that is adapted to calculate feature data of an image captured by the capsule endoscope, and
a determination unit that is adapted to determine whether or not the capsule endoscope has moved from the stomach to the small intestine of the subject based on the feature data calculated by the image processing unit, wherein when the determination unit determines that the capsule endoscope has passed through the stomach, the operation mode setting switches the operation mode of the receiving apparatus to the patient mode.

10. The capsule endoscope system according to claim 8, wherein the control unit (210, 250) is adapted to transmit information about progress of the examination in the receiving apparatus (20) from the receiving apparatus (20) to the control apparatus (30).

11. The capsule endoscope system according to claim 8, wherein the control unit (210, 250) is adapted to display on the display unit (203) information related to a process to be executed or confirmed by the health care professional when the receiving apparatus (20) is set in the health care professional operation mode.

12. The capsule endoscope system according to claim 11, wherein when the operation input unit (204) is adapted to perform operation input in accordance with progress of the examination flow when the receiving apparatus (20) is set in the health care professional operation mode, the control unit (210, 250) permits a shift to a process corresponding to the operation input.

13. The capsule endoscope system according to claim 8, wherein the operation mode setting unit (212) is adapted to switch to the patient operation mode in accordance with progress of the examination flow when the receiving apparatus (20) is set in the health care professional operation mode.

14. The capsule endoscope system according to claim 8, wherein when the operation input unit (204) is adapted to perform operation input other than previously permitted operation input when the receiving apparatus (20) is set in the patient operation mode, the control unit (210, 250) disables the operation input.

15. The capsule endoscope system according to claim 8, wherein the control unit (210, 250) is adapted to display on the display unit (203) information on an instruction to the patient in accordance with progress of the examination flow when the receiving apparatus (20) is set in the patient operation mode.

16. The capsule endoscope system according to claim 8, wherein the operation mode setting unit (212) is adapted to switch the operation mode of the receiving apparatus (20) to the health care professional operation mode when the operation input unit (204) performs a predetermined operation input or when the receiving apparatus (20) receives predetermined control information when the receiving apparatus (20) is set in the patient operation mode.

## Patentansprüche

1. Empfangsvorrichtung (20) zum Empfangen von Information, die drahtlos von einem Kapselendoskop (10) zum Einführen in ein Subjekt und Erfassen eines In-vivo-Bilds des Subjekts gesendet wird, wobei die Empfangsvorrichtung (20) umfasst:
eine Betriebseingabeeinheit (204), die so ausgelegt ist, dass sie Informationseingabe in der Empfangsvorrichtung (20) akzeptiert;
eine Anzeigeeinheit (203), die Information zu einer Untersuchung unter Verwendung des Kapselendoskops (10) anzeigt,
einen Speicher (209), der so ausgelegt ist, dass er Untersuchungsablaufinformation aufzeichnet, die einen Inhalt einer Reihe von Prozessen enthält, die von einem Gesundheitspersonal oder einem Patienten ausgeführt oder bestätigt werden, in den Untersuchungs- und Betriebsmodi des Betreibens der Empfangsvorrichtung (20) durch das Gesundheitspersonal oder den Patienten, um die jeweiligen Prozesse in der Reihe von Prozessen auszuführen oder zu bestätigen; und
eine Steuereinheit (210, 250), die so ausgelegt ist, dass sie Betriebe der Empfangsvorrichtung (20) steuert, wobei die Steuereinheit (210, 250) umfasst:
eine Untersuchungsablaufverwaltungseinheit (211), die so ausgelegt ist, dass sie einen Ablauf einer Reihe von Prozessen bei der Untersuchung auf Basis der im Speicher (209) aufgezeichneten Untersuchungsablaufinformation steuert, **dadurch gekennzeichnet, dass** sie ferner
eine Betriebsmoduseinstelleinheit (212) umfasst, die so ausgelegt ist, dass sie einen Betriebsmodus aus der Untersuchungsablaufinformation extrahiert, der mit einem Prozess in einem aktuellen Untersuchungsablauf assoziiert ist, der von der Untersuchungsablaufverwaltungseinheit (211) gesteuert wird, und
dass sie einen Betriebsmodus der Empfangsvorrichtung (20) zwischen einem Gesundheitspersonal-Betriebsmodus, bei dem das Gesundheitspersonal die Prozesse durch Betreiben der Empfangsvorrichtung (20) ausführt oder bestätigt, und einem Patienten-Betriebsmodus wechselt, in dem der Patient die Prozesse durch Betreiben der Empfangsvorrichtung (20) ausführt oder bestätigt, gemäß dem extrahierten Betriebsmodus, und
dass sie die Betriebe der Empfangsvorrichtung (20) gemäß einem Fortschritt des Untersuchungsablaufs für jeden Betriebsmodus steuert, der von der Betriebsmodussteuereinheit (212) eingestellt wird.

2. Empfangsvorrichtung nach Anspruch 1, wobei die Steuereinheit (210, 250) so ausgelegt ist, dass sie Information zu einem Prozess auf der Anzeigeeinheit (203) anzeigt, der vom Gesundheitspersonal auszuführen oder zu bestätigen ist, wenn die Empfangsvorrichtung (20) im Gesundheitspersonal-Betriebsmodus eingestellt ist.

3. Empfangsvorrichtung nach Anspruch 2, wobei, wenn die Betriebseingabeeinheit (204) so ausgelegt ist, dass sie eine Betriebseingabe gemäß einem Prozess in einem aktuellen Untersuchungsablauf durchführt, der von der Untersuchungsablaufverwaltungseinheit (211) gesteuert wird, wenn die Empfangsvorrichtung (20) im Gesundheitspersonal-Betriebsmodus eingestellt ist, die Untersuchungsablaufverwaltungseinheit einen Wechsel auf einen Prozess entsprechend der Betriebseingabe zulässt.

4. Empfangsvorrichtung nach Anspruch 1, wobei die Betriebsmoduseinstelleinheit (212) so ausgelegt ist, dass sie den Betriebsmodus der Empfangsvorrichtung (20) gemäß einem Prozess in einem aktuellen Untersuchungsablauf, der von der Untersuchungsablaufverwaltungseinheit (211) gesteuert wird, auf den Patienten-Betriebsmodus zu wechseln, wenn die Empfangsvorrichtung (20) im Gesundheitspersonal-Betriebsmodus eingestellt ist.

5. Empfangsvorrichtung nach Anspruch 1, wobei, wenn die Betriebseingabeeinheit (204) so ausgelegt ist, dass sie eine Betriebseingabe durchführt, bei der es sich nicht um die zuvor zugelassene Betriebseingabe handelt, wenn die Empfangsvorrichtung (20) im Patienten-Betriebsmodus eingestellt ist, und die Steuereinheit (201) so ausgelegt ist, dass sie die Betriebseingabe deaktiviert.

6. Empfangsvorrichtung nach Anspruch 1, wobei die Steuereinheit (210, 250) so ausgelegt ist, dass sie Anweisungsinformation für den Patienten auf der Anzeigeeinheit (203) gemäß einem Prozess in einem aktuellen Untersuchungsablauf anzeigt, der von der Untersuchungsablaufverwaltungseinheit (211) gesteuert wird, wenn die Empfangsvorrichtung (20) im Patienten-Betriebsmodus eingestellt ist.

7. Empfangsvorrichtung nach Anspruch 1, wobei die Betriebsmoduseinstelleinheit (212) so ausgelegt ist, dass sie den Betriebsmodus der Empfangsvorrichtung (20) auf den Gesundheitspersonal-Betriebsmodus wechselt, wenn die Betriebseingabeeinheit (204) eine vordefinierte Betriebseingabe durchführt, wenn die Empfangsvorrichtung (20) im Patientenbetriebsmodus eingestellt ist.

8. Kapselendoskopsystem (1), das umfasst:
ein Kapselendoskop (10) zum Einführen in ein Subjekt und Erfassen eines In-vivo-Bilds des Subjekts;
die Empfangsvorrichtung (20) nach Anspruch 1; und
eine Steuervorrichtung (30) zum Senden und Empfangen von Information in einer kabelgebundenen oder drahtlosen Kommunikation an die Empfangsvorrichtung (20) bzw. von dieser.

9. Kapselendoskopsystem nach Anspruch 8, das ferner umfasst:
eine Bildverarbeitungseinheit, die so ausgelegt ist, dass sie Merkmalsdaten eines Bilds berechnet, das vom Kapselendoskop erfasst wird, und
eine Ermittlungseinheit, die so ausgelegt ist, dass sie ermittelt, ob sich das Kapselendoskop vom Magen in den Dünndarm des Subjekts bewegt hat, auf Basis der Merkmalsdaten, die von der Bildverarbeitungseinheit berechnet werden, wobei, wenn die Ermittlungseinheit ermittelt, dass das Kapselendoskop den Magen passiert hat, die Betriebsmoduseinstellung den Betriebsmodus der Empfangsvorrichtung auf den Patientenmodus wechselt.

10. Kapselendoskopsystem nach Anspruch 8, wobei die Steuereinheit (210, 250) so ausgelegt ist, dass sie eine Information zu einem Fortschritt der Untersuchung in der Empfangsvorrichtung (20) von der Empfangsvorrichtung (20) an die Steuervorrichtung (30) sendet.

11. Kapselendoskopsystem nach Anspruch 8, wobei die Steuereinheit (210, 250) so ausgelegt ist, dass sie Information zu einem Prozess, der von Gesundheitspersonal durchzuführen oder zu bestätigen ist, wenn die Empfangsvorrichtung (20) im Gesundheitspersonal-Betriebsmodus eingestellt ist, auf der Anzeigeeinheit (203) anzeigt.

12. Kapselendoskopsystem nach Anspruch 11, wobei, wenn die Betriebseingabeeinheit (204) so ausgelegt ist, dass sie eine Betriebseingabe gemäß einem Fortschritt des Untersuchungsablaufs durchführt, wenn die Empfangsvorrichtung (20) im Gesundheitspersonal-Betriebsmodus eingestellt ist, die Steuereinheit (210, 250) einen Wechsel auf einen Prozess entsprechend der Betriebseingabe zulässt.

13. Kapselendoskopsystem nach Anspruch 8, wobei die Betriebsmoduseinstelleinheit (212) so ausgelegt ist, dass sie gemäß einem Fortschritt des Untersuchungsablaufs auf den Patienten-Betriebsmodus wechselt, wenn die Empfangsvorrichtung (20) im Gesundheitspersonal-Betriebsmodus eingestellt ist.

14. Kapselendoskopsystem nach Anspruch 8, wobei, wenn die Betriebseingabeeinheit (204) so ausgelegt ist, dass sie eine Betriebseingabe durchführt, bei der es sich nicht um die zuvor zugelassene Betriebseingabe handelt, wenn die Empfangsvorrichtung (20) im Patienten-Betriebsmodus eingestellt ist, die Steuereinheit (210, 250) die Betriebseingabe deaktiviert.

15. Kapselendoskopsystem nach Anspruch 8, wobei die Steuereinheit (210, 250) so ausgelegt ist, dass sie eine Information zu einer Anweisung für den Patienten gemäß einem Fortschritt des Untersuchungsablaufs, wenn die Empfangsvorrichtung (20) im Patienten-Betriebsmodus eingestellt ist, auf der Anzeigeeinheit (203) anzeigt.

16. Kapselendoskopsystem nach Anspruch 8, wobei die Betriebsmoduseinstelleinheit (212) so ausgelegt ist, dass sie den Betriebsmodus der Empfangsvorrichtung (20) auf den Gesundheitspersonal-Betriebsmodus wechselt, wenn die Betriebseingabeeinheit (204) eine vordefinierte Betriebseingabe durchführt, oder wenn die Empfangsvorrichtung (20) vordefinierte Steuerinformation empfängt, wenn die Empfangsvorrichtung (20) im Patienten-Betriebsmodus eingestellt ist.

## Revendications

1. Appareil de réception (20) pour recevoir des informations émises de manière sans fil à partir d'une capsule endoscopique (10) pour insertion dans un sujet et capturer une image in vivo du sujet, l'appareil de réception (20) comprenant :
une unité d'entrée d'opération (204) qui est adaptée pour accepter une entrée d'informations dans l'appareil de réception (20) ;
une unité d'affichage (203) qui affiche des informations relatives à un examen utilisant la capsule endoscopique (10),
une mémoire (209) qui est adaptée pour enregistrer des information de flux d'examen contenant des contenus d'une série de traitements qui sont exécutés ou confirmés par un professionnel de santé ou un patient dans l'examen et des modes opérationnels d'opération de l'appareil de réception (20) par le professionnel de santé ou le patient pour exécuter ou confirmer les traitements respectifs dans la série de traitements ; et
une unité de commande (210, 250) qui est adaptée pour commander des opérations de l'appareil de réception (20), l'unité de commande (210, 250) comprenant :
une unité de gestion de flux d'examen (211) qui est adaptée pour commander un flux d'une série de traitements dans l'examen sur la base des informations de flux d'examen enregistrées dans la mémoire (209), **caractérisé par le fait qu'**il comprend en outre
une unité de réglage de mode opérationnel (212) qui est adaptée pour extraire des informations de flux d'examen un mode opérationnel associé à un traitement dans un flux d'examen actuel commandé par l'unité de gestion de flux d'examen (211), et
pour commuter un mode opérationnel de l'appareil de réception (20) entre un mode opérationnel de professionnel de santé dans lequel le professionnel de santé exécute ou confirme les traitements en actionnant l'appareil de réception (20) et un mode opérationnel de patient dans lequel le patient exécute ou confirme les traitements en actionnant l'appareil de réception (20) conformément au mode opérationnel extrait, et
pour commander les opérations de l'appareil de réception (20) conformément à une progression du flux d'examen pour chaque mode opérationnel réglé par l'unité de réglage de mode opérationnel (212).

2. Appareil de réception selon la revendication 1, dans lequel l'unité de commande (210, 250) est adaptée pour afficher sur l'unité d'affichage (203) des informations relatives à un traitement à exécuter ou à confirmer par le professionnel de santé lorsque l'appareil de réception (20) est réglé dans le mode opérationnel de professionnel de santé.

3. Appareil de réception selon la revendication 2, dans lequel, lorsque l'unité d'entrée d'opération (204) est adaptée pour effectuer une entrée d'opération conformément à un traitement dans un flux d'examen actuel commandé par l'unité de gestion de flux d'examen (211) lorsque l'appareil de réception (20) est réglé dans le mode opérationnel de professionnel de santé, l'unité de gestion de flux d'examen permet un passage à un traitement correspondant à l'entrée d'opération.

4. Appareil de réception selon la revendication 1, dans lequel l'unité de réglage de mode opérationnel (212) est adaptée pour commuter le mode opérationnel de l'appareil de réception (20) vers le mode opérationnel de patient conformément à un traitement dans un flux d'examen actuel commandé par l'unité de gestion de flux d'examen (211) lorsque l'appareil de réception (20) est réglé dans le mode opérationnel de professionnel de santé.

5. Appareil de réception selon la revendication 1, dans lequel, lorsque l'unité d'entrée d'opération (204) est adaptée pour effectuer une entrée d'opération autre qu'une entrée d'opération précédemment permise lorsque l'appareil de réception (20) est réglé dans le mode opérationnel de patient, et l'unité de commande (201) est adaptée pour désactiver l'entrée d'opération.

6. Appareil de réception selon la revendication 1, dans lequel l'unité de commande (210, 250) est adaptée pour afficher des informations d'instruction au patient sur l'unité d'affichage (203) conformément à un traitement dans un flux d'examen actuel commandé par l'unité de gestion de flux d'examen (211) lorsque l'appareil de réception (20) est réglé dans le mode opérationnel de patient.

7. Appareil de réception selon la revendication 1, dans lequel l'unité de réglage de mode opérationnel (212) est adaptée pour commuter le mode opérationnel de l'appareil de réception (20) vers le mode opérationnel de professionnel de santé lorsque l'unité d'entrée d'opération (204) effectue une entrée d'opération prédéterminée lorsque l'appareil de réception (20) est réglé dans le mode opérationnel de patient.

8. Système de capsule endoscopique (1) comprenant :
une capsule endoscopique (10) destinée à être insérée dans un sujet et à capturer une image in vivo du sujet ;
l'appareil de réception (20) selon la revendication 1 ; et
un appareil de commande (30) pour émettre et recevoir des informations en communication filaire ou sans fil vers et depuis l'appareil de réception (20).

9. Système de capsule endoscopique selon la revendication 8, comprenant en outre :
une unité de traitement d'image qui est adaptée pour calculer des données caractéristiques d'une image capturée par la capsule endoscopique, et
une unité de détermination qui est adaptée pour déterminer si oui ou non la capsule endoscopique est passée de l'estomac à l'intestin grêle du sujet sur la base des données caractéristiques calculées par l'unité de traitement d'image, dans lequel lorsque l'unité de détermination détermine que la capsule endoscopique est passée à travers l'estomac, l'unité de réglage de mode opérationnel commute le mode opérationnel de l'appareil de réception vers le mode de patient.

10. Système de capsule endoscopique selon la revendication 8, dans lequel l'unité de commande (210, 250) est adaptée pour émettre des informations concernant une progression de l'examen dans l'appareil de réception (20) depuis l'appareil de réception (20) vers l'appareil de commande (30).

11. Système de capsule endoscopique selon la revendication 8, dans lequel l'unité de commande (210, 250) est adaptée pour afficher sur l'unité d'affichage (203) des informations relatives à un traitement à exécuter ou à confirmer par le professionnel de santé lorsque l'appareil de réception (20) est réglé dans le mode opérationnel de professionnel de santé.

12. Système de capsule endoscopique selon la revendication 11, dans lequel lorsque l'unité d'entrée d'opération (204) est adaptée pour effectuer une entrée d'opération conformément à une progression du flux d'examen lorsque l'appareil de réception (20) est réglé dans le mode opérationnel de professionnel de santé, l'unité de commande (210, 250) permet un passage à un traitement correspondant à l'entrée d'opération.

13. Système de capsule endoscopique selon la revendication 8, dans lequel l'unité de réglage de mode opérationnel (212) est adaptée pour commuter vers le mode opérationnel de patient conformément à une progression du flux d'examen lorsque l'appareil de réception (20) est réglé dans le mode opérationnel de professionnel de santé.

14. Système de capsule endoscopique selon la revendication 8, dans lequel lorsque l'unité d'entrée d'opération (204) est adaptée pour effectuer une entrée d'opération autre qu'une entrée d'opération précédemment permise lorsque l'appareil de réception (20) est réglé dans le mode opérationnel de patient, l'unité de commande (210, 250) désactive l'entrée d'opération.

15. Système de capsule endoscopique selon la revendication 8, dans lequel l'unité de commande (210, 250) est adaptée pour afficher sur l'unité d'affichage (203) des informations sur une instruction au patient conformément à une progression du flux d'examen lorsque l'appareil de réception (20) est réglé dans le mode opérationnel de patient.

16. Système de capsule endoscopique selon la revendication 8, dans lequel l'unité de réglage de mode opérationnel (212) est adaptée pour commuter le mode opérationnel de l'appareil de réception (20) vers le mode opérationnel de professionnel de santé lorsque l'unité d'entrée d'opération (204) effectue une entrée d'opération prédéterminée ou lorsque l'appareil de réception (20) reçoit des information de commande prédéterminées lorsque l'appareil de réception (20) est réglé dans le mode opérationnel de patient.
